# EUROPEAN PATENT APPLICATION

(11) **EP 4 360 624 A1**
(43) Date of publication of application: **01.05.2024**
(21) Application number: 22828788.4
(22) Date of filing: 23.06.2022
(51) Int. Cl.: A61K 9/51, A61K 47/18, A61K 47/26, A61K 47/28, A61K 47/24, A61K 31/7088, A61K 47/10, A61K 48/00

(54) **LIPID NANOPARTICLES AND METHOD FOR PREPARING SAME**

(30) Priority: 24.06.2021 KR 20210082361; 22.06.2022 KR 20220076212
(71) Applicant: Therna Therapeutics, Seoul 07573 (KR)
(72) Inventor: LEE, Taewoo, Seoul 06923 (KR); BAEK, Yu Mi, Seoul 05075 (KR); RYU, Jiwon, Seoul 07602 (KR); JANG, Dahye, Bucheon-si Gyeonggi-do 14424 (KR)
(74) Representative: Mewburn Ellis LLP
(86) International application number: PCT/KR2022/008941
(87) International publication number: WO 2022/270941

(57) **Abstract**

The present invention relates to lipid nanoparticles and a method for preparing same and, more specifically, to particles containing an ionizable lipid and a polyethylene glycol moiety (PEG moiety)-degradable liking functional group-lipid conjugate, which are characterized by minimized side effects in vivo and effective nanoparticle transfer into target cells so as to deliver pharmaceutically effective materials into the cytoplasm.

## Description

### [Technical Field]

The present invention relates to lipid nanoparticles and a method of producing the same, and more specifically, to lipid nanoparticles that contain an ionizable lipid and a polyethylene glycol derivative moiety (PEG moiety)-degradable linking functional group-lipid conjugate, thus minimizing side effects *in vivo,* effectively delivering nanoparticles to target cells, and efficiently transporting pharmacological substances into the cytoplasm and a method of producing the same.

### [Background Art]

The efficiencies of a drug greatly vary depending on the delivery method thereof. A drug system that has an administration route to efficiently deliver the required amount of drug by minimizing the side effects of the drug while maximizing the efficacy and effectiveness thereof is referred to as a "drug delivery system (DDS)". In the pharmaceutical formulation industry, drug delivery systems are considered high-value core technology that has high success potential and provides economic benefits comparable to development of novel drugs.

For example, solubilization of poorly-soluble drugs that belongs to drug absorption facilitation, which is a key technology in drug delivery systems, is considered one of the most reasonable methods to reduce the development cost of novel drug substances and increase the added value of pharmaceuticals. In particular, the development of improved novel drugs based on the development of drug solubilization technologies can create enormous added value at a low cost.

Recently, the development of gene therapy using genetic drug delivery systems among various drug delivery systems has gradually expanded. In order to perform gene therapy successfully and safely, it is important to deliver genes or gene regulators to desired tissues. Representative examples of such genes or gene regulators include mRNA and siRNA. Since nucleic acids such as mRNA function to express specific proteins, they can compensate for the loss of proteins due to genetic factors. In addition, it is possible to develop anticancer drugs and vaccines that activate the immune response *in vivo* using mRNA that expresses cancer markers and viral surface proteins. In addition, nucleic acids such as siRNA are substances that can inhibit the expression of specific proteins *in vivo,* and are attracting attention as a key tool in the treatment of cancer, genetic diseases, infectious diseases, and autoimmune diseases. However, it is difficult to deliver nucleic acids such as mRNA and siRNA into cells, per se, and nucleic acids are easily degraded by enzymes in the blood. Therefore, research is being actively conducted to overcome these problems.

### Characteristics and limitations of cationic liposomes or cationic polymer-based nanoparticles

To effectively deliver these substances, non-viral gene carriers such as cationic liposomes and polymers have been developed and the improved stability profile and ease of fabrication and manipulation of polymer carriers have led to accelerated research on design and synthesis of non-toxic and biodegradable polymer carriers. Poly(L-lysine), polyethylenimine, starburst, polyamidoamine dendrimers, cationic liposomes and the like can spontaneously self-assemble with anionic oligomers and compress plasmid DNA (pDNA) into structures small enough to introduce plasmid DNA (pDNA) into cells through endocytosis, thus being widely studied as non-viral gene carriers.

However, nanoparticles produced using a method based on cationic liposomes or cationic polymers have limitations unsuitable for development into therapeutic agents. The method is characterized in that cationic liposomes or polymers are mixed with anionic oligos to produce nanoparticles through electrostatic attraction. Therefore, an excessive amount of cationic material must be used compared to the amount of anionic charge in order to produce stable particles. Since nanoparticles cannot be formed when the same molar ratio of cations and anions is used, cations are used in an excess amount, more specifically, at least several to tens of times. Therefore, the cations still exist in excess in the nanoparticle even after part of them are used to interact with anions and neutralized when nanoparticles are produced in this way. Therefore, the produced nanoparticles constantly maintain cationic charges. However, it is widely known that positively charged nanoparticles have the disadvantage of causing side effects due to unnecessary interactions with various cells, including but not limited to immune cells, *in vivo.* Therefore, it is difficult to develop therapeutic agents using these types of nanoparticles.

### Characteristics of lipid nanoparticles using ionizable lipids and methods of producing the same

Recently, an effective therapeutic agent has been developed using a new type of lipid nanoparticle as a non-viral gene carrier that overcomes the disadvantages of cationic nanoparticles (Non-Patent Documents 1-9). These lipid nanoparticles are particulate drug carriers that are body-friendly because they use substances present in the living body, such as phospholipids and cholesterol, enable drug release and control, and are highly stable against degradation by enzymes, and the like. In particular, the essential difference between such lipid nanoparticles and conventional cationic nanoparticles is that the lipid nanoparticles contain ionizable lipids as a component. Ionizable lipids are characterized in that they are cationic at an acidic pH, but remain electrically neutral at neutral pH. Therefore, nanoparticles produced using ionizable lipids are electrically neutral in the bloodstream of which pH is neutral so that they can dramatically eliminate side effects *in vivo* caused by the cationic nature of cationic polymers or cationic liposomes.

Another difference therebetween relates to a method of producing lipid nanoparticles. More specifically, the method of producing lipid nanoparticles is described as follows. Lipid nanoparticles are produced by mixing lipid components dissolved in an organic phase with oligos dissolved in an aqueous buffer solution. Because the polarity of the solvent changes as the organic phase is mixed with the aqueous solution, the lipid component contained in the organic phase spontaneously forms particles in the mixed solution. At this time, when the ionizable lipid contained in the organic phase is mixed with an aqueous buffer solution containing oligos under acidic conditions, the ionizable lipids become cationic due to the acidic conditions, and the cationic ionizable lipids are bound to the anionic oligos by electrostatic attraction to produce nanoparticles containing oligos.

Another characteristic of lipid nanoparticles employing ionizable lipids is that some lipid components which can function to prevent aggregation of nanoparticles during the process of particle formation should be included in the organic phase. Lipids suitable for preventing aggregation of nanoparticles have a molecular structure in which a lipid is linked to a hydrophilic substance. The hydrophilic substance may be a biological substance such as carbohydrate or protein or may be a synthetic substance such as polyol and polyethylene glycol (PEG). A representative example thereof is a PEG-lipid conjugate. In the PEG-lipid conjugate, the lipid is hydrophobic and is thus directed to the inside of the particle when lipid nanoparticles are formed, whereas the PEG is hydrophilic and thus is located on the surface of the lipid nanoparticles and is directed to the outside of the particle (non-patent Documents 11-12). Unless, in this process, the lipid for preventing particle aggregation, such as PEG-lipid conjugate, constituting the surface of the nanoparticle, is contained as a component, the nanoparticles may continuously aggregate with each other. As a consequence, it is very difficult to produce nanoparticles with a relatively uniform size and it is also difficult to expect efficacy from non-uniform nanoparticles.

Another reason why PEG is typically used as a hydrophilic substance of the lipid for preventing aggregation is that PEG constituting the surface of lipid nanoparticles also affects the biological properties of lipid nanoparticles. In general, nanoparticles administered into the body disadvantageously are lost due to phagocytosis by macrophages through adsorption to proteins, lipids, and immune components in the bloodstream. In order to solve this problem, it is known that this disadvantage can be overcome by modifying the surface of nanoparticles with PEG, a hydrophilic polymer having excellent biocompatibility (US Patent No. 9,999,673 B2; KR Patent Laid-open No. 10-2018-0032652, WO 2020/061284). PEG is most commonly used for this purpose because it has no side effects on the living body and is easily applied to lipid nanoparticles and other drug delivery systems (Non-patent Document 10).

Disadvantages of use of PEG-lipid conjugates and limitations of conventional solutions.

PEG has fundamental limitations although it is an essential component in the process of producing lipid nanoparticles and has a remarkable advantage of reducing many side effects by suppressing non-specific interactions of nanoparticles with other substances *in vivo.* The fact that PEG reduces non-specific interactions between nanoparticles and other biological components *in vivo* ultimately results in the inhibition of the interaction between nanoparticles and target cells. Therefore, PEG makes it difficult to deliver nanoparticles into target cells, and as a result, pharmacological efficiency is remarkably reduced (Non-Patent Documents 13-15).

In an attempt to solve the problem caused by the fact that PEG inhibits the interaction between nanoparticles and target cells, two methods were designed. One is a method of separating PEG from lipid nanoparticles after the nanoparticles are administered into the body. Generally, the lipid part in the PEG-lipid conjugate is composed of two strands of fatty acid. When the hydrophobicity of the fatty acid is reduced by decreasing the number of carbons constituting the fatty acid, the PEG-lipid conjugate is easily separated and removed from the nanoparticles while the nanoparticles circulate *in vivo.* As a result, the efficiency of the nanoparticles can be maintained because the interference effect caused by PEG is eliminated. It is known that, when the number of carbons constituting the fatty acid increases, the PEG-lipid conjugate is not removed from the nanoparticles and the efficacy thereof decreases (Non-Patent Documents 14-16). However, when the PEG-lipid conjugate, which is a component of the nanoparticle, is removed in such a manner, disadvantageously, the particles become unstable and other components constituting the particles are also lost (Non-patent Document 17).

The other method to solve the problem of the decreased delivery efficiency of lipid nanoparticles to target cells due to PEG is to conjugate a ligand capable of binding to a cell receptor at the end of PEG (Non-patent Document 16). Because the ligand at the end of PEG binds to the receptor of a specific cell, the nanoparticles containing the ligand can be delivered to endosomes within the target cells by the receptor. However, even if nanoparticles can be effectively delivered to endosomes within cells using ligands, nucleic acids, which are pharmacological substances contained in the nanoparticles, must be delivered from the endosomes into the cytoplasm in order to obtain pharmacological efficacy. For this purpose, a fusion process is required through interaction between the lipid nanoparticles and the endosomal lipid membrane, but PEG surrounding the outside of the nanoparticles interferes with this function. As a result, attaching a ligand to the end of PEG enables nanoparticles to be delivered into the endosomes of target cells, but disables the therapeutic substance to be delivered from the endosome to the cytoplasm. Therefore, this method cannot remarkably improve overall efficiencies (Non-patent Document 16).

### [22] Application of PEG-degradable functional group-lipid conjugate to ionizable lipid nanoparticles

In order to solve the decrease in efficacy caused by PEG in endosomes, a method of removing the PEG surrounding the surface of lipid particles in endosomes is usually used. In this case, a material in the form of a PEG-degradable functional group-lipid conjugate is contained as a component such that the site linking the PEG to the lipid has a functional group that can be hydrolyzed under acidic conditions since the pH inside the endosome is acidic. As a result, PEG, which constitutes the surface of nanoparticles within endosomes, can be effectively removed (Non-Patent Documents 18-19).

However, the conditions for producing nanoparticles are inevitably unfavorable for PEG-degradable functional group-lipid conjugate-type material to be used as a component since itcontains the functional linker that degrades under acidic conditions. Because the degradable functional group of the PEG-degradable functional group-lipid conjugate is unstable under acidic conditions, the material can be applied only to nanoparticles produced under neutral pH or alkaline conditions. However, lipid nanoparticles, which are widely used in the recent development of various oligo-based therapeutics, contain ionizable lipids as a component, and as described above, lipid nanoparticles containing oligo components using ionizable lipids must be produced at an acidic pH. For this reason, when producing lipid nanoparticles containing ionizable lipids, using a component having a functional group that is hydrolyzed under acidic conditions, that is, a PEG-degradable functional group-lipid conjugate, as a component, has been considered essentially impossible and never been attempted to date.

Accordingly, as a result of extended efforts to solve the problem, the present inventors found that lipid nanoparticles containing an ionizable lipid can be stably produced using a polyethylene glycol moiety (PEG moiety)-degradable functional group-lipid conjugate. In particular, the PEG moiety-degradable functional group-lipid conjugate remains stable under the acidic conditions of the process for producing lipid nanoparticles, but is effectively hydrolyzed in the acidic conditions of endosomes, facilitating fusion of the endosomal lipid membrane and lipid nanoparticles and remarkably promoting oligo delivery efficiency. The present inventors found that, when these lipid nanoparticles are produced and used as a therapeutic agent, they can minimize side effects *in vivo* caused by the components of the nanoparticles, effectively transfer the nanoparticles to target cells, and are suitable for effectively delivering pharmacological substances such as nucleic acids from the endosomes to the cytoplasm in the cells. Based thereon, the present invention has been completed.

### [Prior art Document]

### [Patent Document]

US Patent No. 9,999,673 B2

KR Patent Publication No. 10-2018-0032652

International Publication WO 2020/061284

### [Non-patent Document]

1. Moss, K.H., Popova, P., Hadrup, S.R., Astakhova, K. & Taskova, M. Lipid Nanoparticles for Delivery of Therapeutic RNA Oligonucleotides. Mol Pharm 16, 2265-2277 (2019).
2. Kulkarni, J.A., Witzigmann, D., Chen, S., Cullis, P.R. & van der Meel, R. Lipid Nanoparticle Technology for Clinical Translation of siRNA Therapeutics. Acc Chem Res 52, 2435-2444 (2019).
3. Buck, J., Grossen, P., Cullis, P.R., Huwyler, J. & Witzigmann, D. Lipid-Based DNA Therapeutics: Hallmarks of Non-Viral Gene Delivery. ACS Nano 13, 3754-3782 (2019).
4. Akinc, A. et al. The Onpattro story and the clinical translation of nanomedicines containing nucleic acid-based drugs. Nat Nanotechnol 14, 1084-1087 (2019).
5. Springer, A.D. & Dowdy, S.F. GalNAc-siRNA Conjugates: Leading the Way for Delivery of RNAi Therapeutics. Nucleic Acid Ther 28, 109-118 (2018).
6. Kulkarni, J.A., Cullis, P.R. & van der Meel, R. Lipid Nanoparticles Enabling Gene Therapies: From Concepts to Clinical Utility. Nucleic Acid Ther 28, 146-157 (2018).
7. Rietwyk, S. & Peer, D. Next-Generation Lipids in RNA Interference Therapeutics. ACS Nano 11, 7572-7586 (2017).
8. Fang, Y. et al. Cleavable PEGylation: a strategy for overcoming the "PEG dilemma" in efficient drug delivery. Drug Deliv 24, 22-32 (2017).
9. Cullis, P.R. & Hope, M.J. Lipid Nanoparticle Systems for Enabling Gene Therapies. Mol Ther 25, 1467-1475 (2017).
10. Kumar, V. et al. Shielding of Lipid Nanoparticles for siRNA Delivery: Impact on Physicochemical Properties, Cytokine Induction, and Efficacy. Mol Ther Nucleic Acids 3, e210 (2014).
11. Belliveau, N.M. et al. Microfluidic Synthesis of Highly Potent Limit-size Lipid Nanoparticles for In Vivo Delivery of siRNA. Mol Ther Nucleic Acids 1, e37 (2012).
12. Sato, Y. et al. Elucidation of the physicochemical properties and potency of siRNA-loaded small-sized lipid nanoparticles for siRNA delivery. J Control Release 229, 48-57 (2016).
13. Bao, Y. et al. Effect of PEGylation on biodistribution and gene silencing of siRNA/lipid nanoparticle complexes. Pharm Res 30, 342-51 (2013).
14. Mui, B.L. et al. Influence of Polyethylene Glycol Lipid Desorption Rates on Pharmacokinetics and Pharmacodynamics of siRNA Lipid Nanoparticles. Mol Ther Nucleic Acids 2, e139 (2013).
15. Chen, S. et al. Development of lipid nanoparticle formulations of siRNA for hepatocyte gene silencing following subcutaneous administration. J Control Release 196, 106-12 (2014).
16. Akinc, A. et al. Targeted delivery of RNAi therapeutics with endogenous and exogenous ligand-based mechanisms. Mol Ther 18, 1357-64 (2010).
17. Chen, S. et al. Influence of particle size on the in vivo potency of lipid nanoparticle formulations of siRNA. J Control Release 235, 236-244 (2016).
18. Bargh, J.D., Isidro-Llobet, A., Parker, J.S. & Spring, D.R. Cleavable linkers in antibody-drug conjugates. Chem Soc Rev 48, 4361-4374 (2019).
19. Zhao, G. et al. Smart pH-sensitive nanoassemblies with cleavable PEGylation for tumor targeted drug delivery. Sci Rep 7, 3383 (2017).

### [Disclosure]

### [Technical Problem]

Therefore, the present invention has been made in view of the above problems, and it is an object of the present invention to provide lipid nanoparticles that minimize side effects *in vivo* caused by the components of nanoparticles, effectively deliver nanoparticles to target cells, and efficiently transport pharmacological substances such as nucleic acids from endosomes into the cytoplasm in target cells.

### [Technical Solution]

In accordance with one aspect of the present invention, the above and other objects can be accomplished by the provision of a lipid nanoparticle including: (a) a lipid formulations containing an ionizable lipid and a polyethylene glycol moiety (PEG moiety)-degradable functional group-lipid conjugate; and (b) a drug, nucleic acid, or combination thereof encapsulated in the lipid formulations.

In accordance with another aspect of the present invention, provided is a method of producing the lipid nanoparticle including (a) mixing an organic solution of a lipid formulations containing an ionizable lipid and a polyethylene glycol moiety (PEG moiety)-degradable functional group-lipid conjugate, with a buffer solution containing a drug, nucleic acid, or combination thereof and adjusting pH, and (b) removing the solvent from the solution.

### [Description of Drawings]

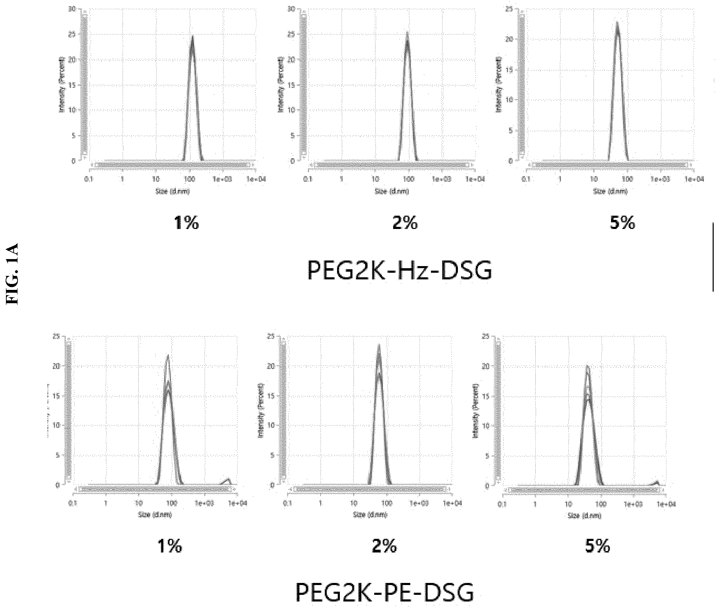

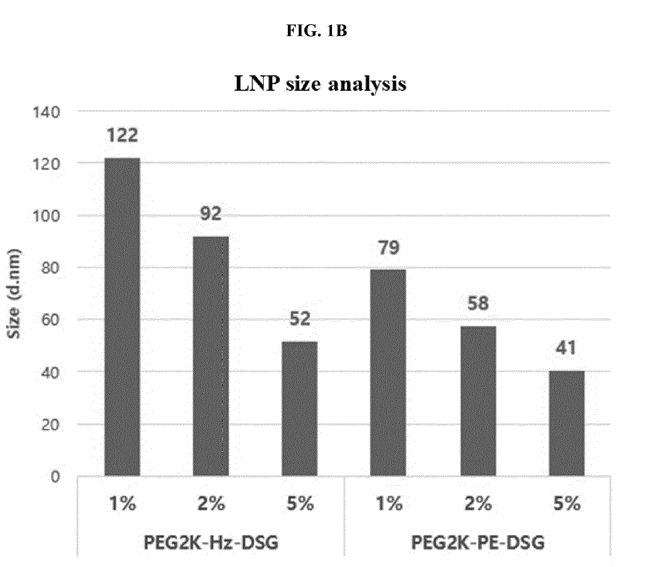

FIG. 1 shows a graph (A) showing the size measured by DLS of lipid nanoparticles containing 1, 2, and 5 mol% of PEG2K-Hz-DSG or PEG2K-PE-DSG based on the total lipid content of the nanoparticles according to an embodiment of the present invention, and a graph (B) showing the quantification result thereof.

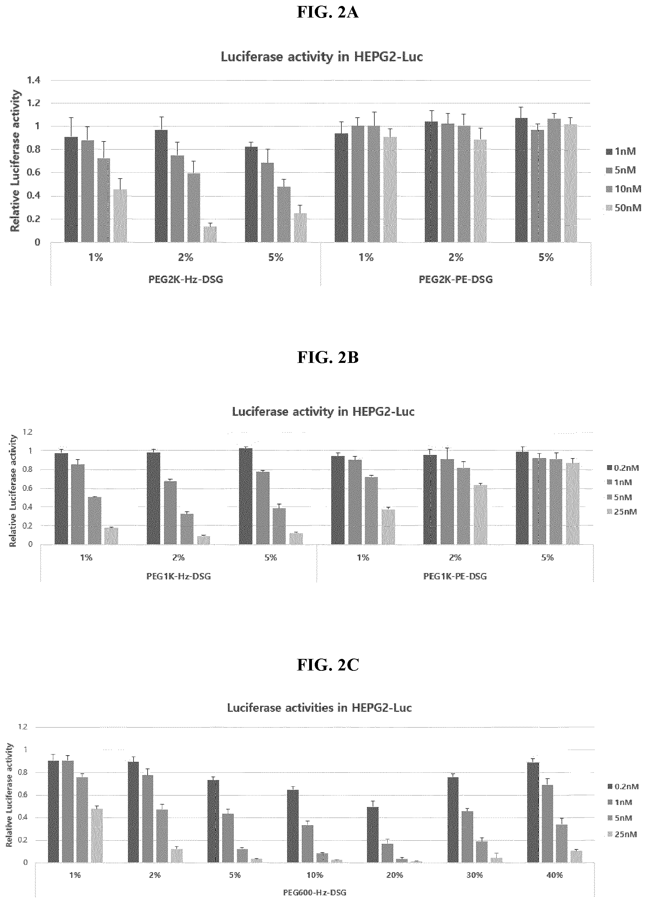

FIG. 2 is a graph showing the efficacy of inhibiting luciferase expression when treating cells constitutively expressing luciferase with lipid nanoparticles loaded with siRNA targeting luciferase according to an embodiment of the present invention, wherein A is a graph showing lipid nanoparticles containing 1, 2, and 5 mol% of PEG2K-Hz-DSG or PEG2K-PE-DSG, B is a graph showing lipid nanoparticles containing 1, 2, and 5 mol% of PEG1K-Hz-DSG or PEG1K-PE-DSG, and C is a graph showing lipid nanoparticles containing 1, 2, 5, 10, 20, 30, or 40 mol% of PEG600-Hz-DSG.

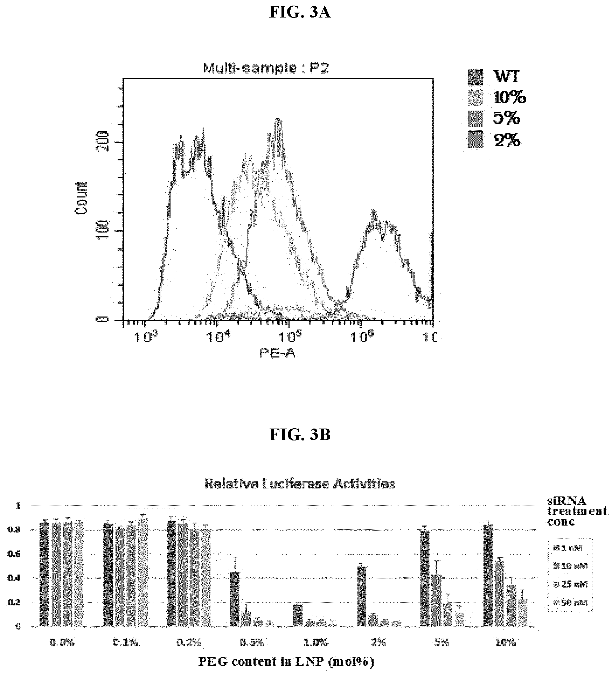

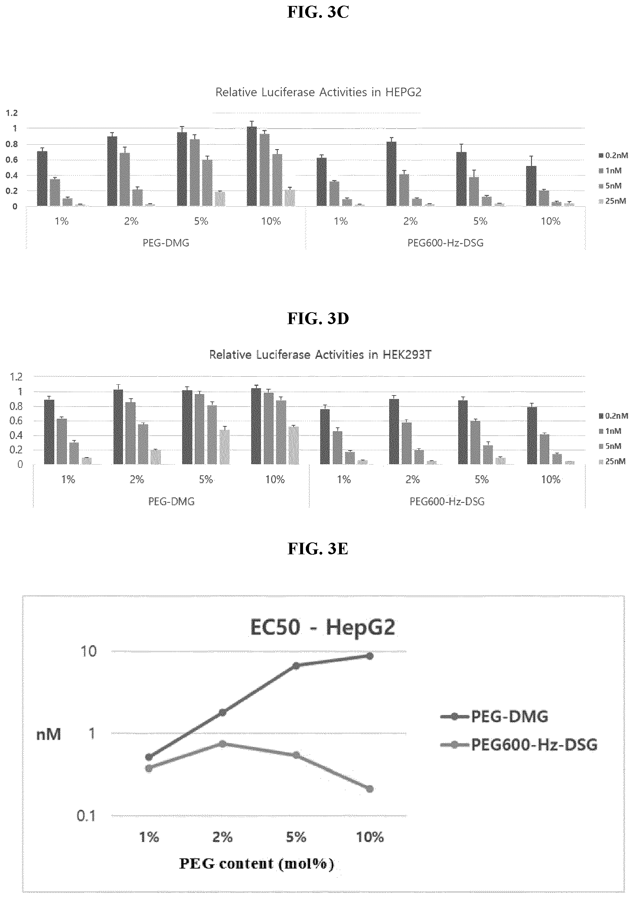

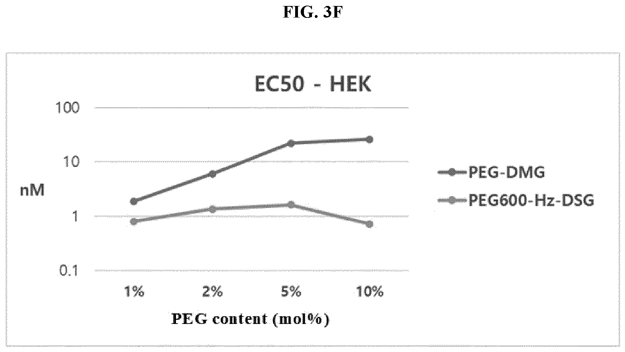

FIG. 3 illustrates the effect of the content of PEG in lipid nanoparticles on the uptake efficiency of cells according to an embodiment of the present invention, wherein A shows cellular uptake efficiency of lipid nanoparticles containing PEG-DMG at 2, 5, and 10 mol% based on the total lipid content analyzed by flow cytometry (FACS), B is a graph showing the efficacy of inhibiting luciferase expression when treating cells constitutively expressing luciferase with lipid nanoparticles loaded with siRNA targeting luciferase containing 0, 0.1, 0.2, 0.5, 1, 2, 5, or 10 mol% of PEG-DMG according to an embodiment of the present invention, and C and D are graphs showing the efficacy of inhibiting luciferase expression in different cell lines by lipid nanoparticles containing 1, 2, 5, and 10 mol% of PEG-DMG or PEG600-Hz-DSG, respectively, and E and F show the data of EC₅₀.

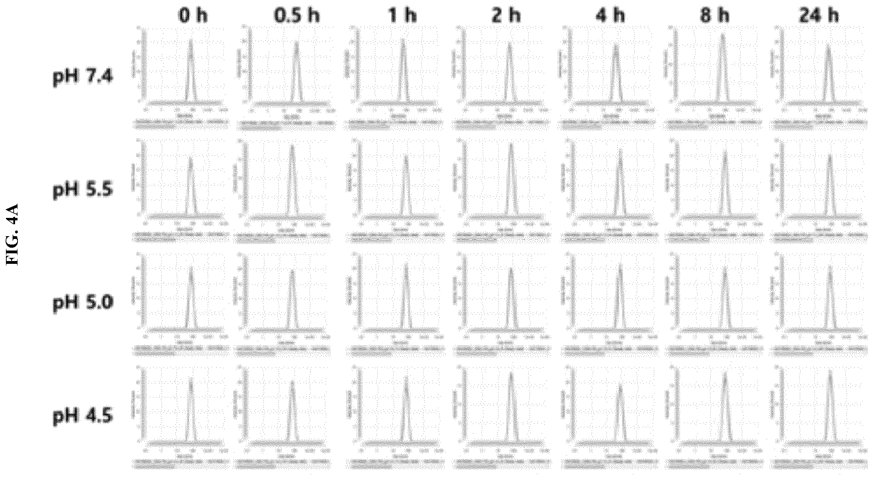

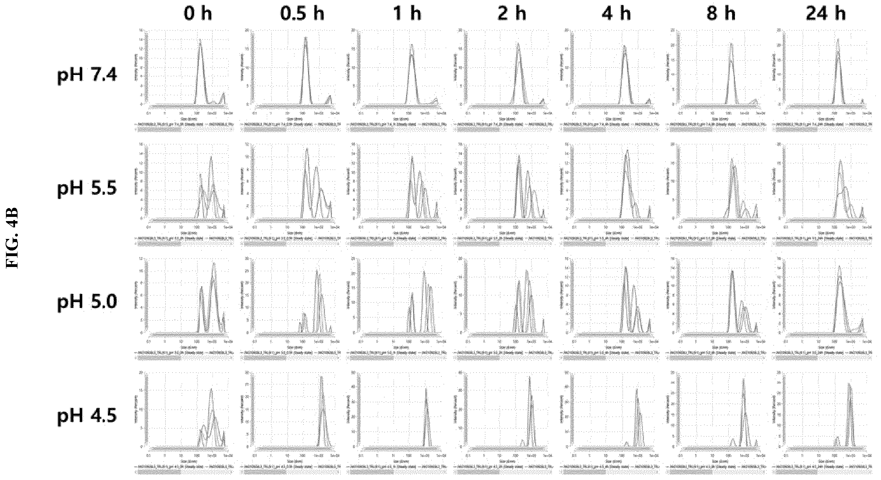

FIG. 4 shows data of the size of lipid nanoparticles containing 1.5 mol% of PEG-DMG (A) or 10 mol% of PEG600-Hz-DSG (B) over time under acidic conditions according to an embodiment of the present invention.

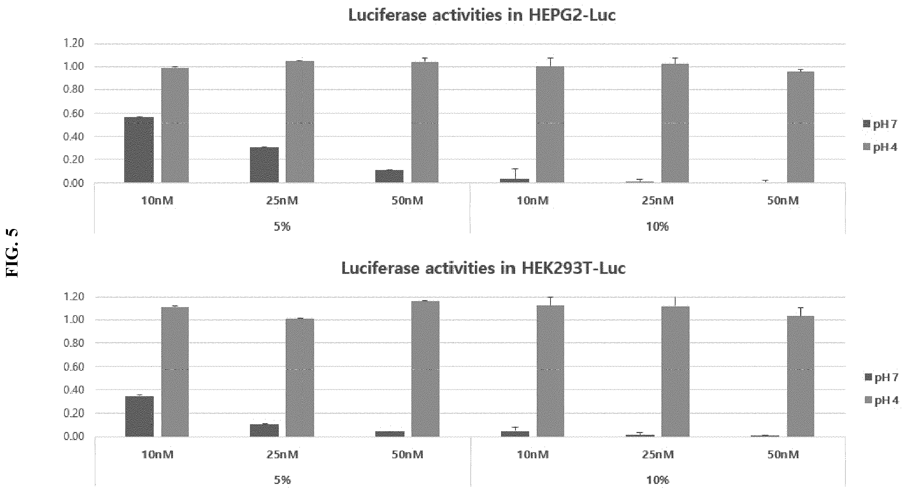

FIG. 5 is a graph showing the efficacy of inhibiting luciferase expression of lipid nanoparticles containing 5 or 10 mol% of PEG600-Hz-DSG according to an embodiment of the present invention upon pretreatment of the lipid nanoparticles at pH 4 or 7, and then treatment of cell lines with the lipid nanoparticles.

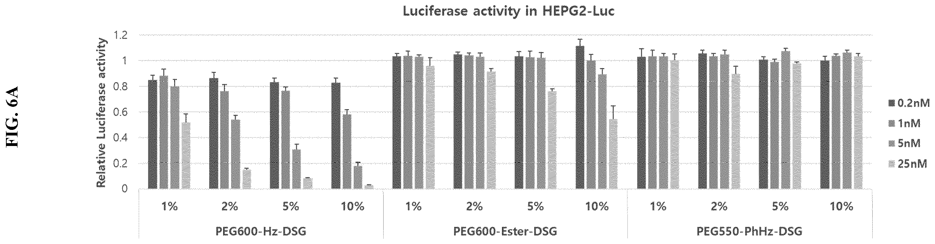

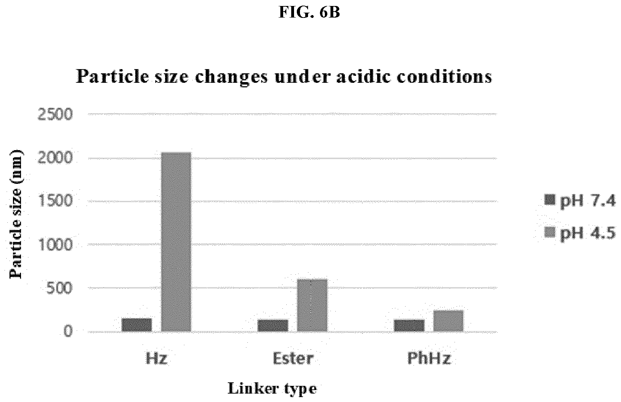

FIG. 6 is a graph showing the efficacy of inhibiting luciferase expression of lipid nanoparticles containing 1, 2, 5, or 10 mol% of PEG600-Hz-DSG, PEG600-Ester-DSG, or PEG600-PhHz-DSG according to an embodiment of the present invention upon treatment of cell lines with the lipid nanoparticles (A) and data of the size of lipid nanoparticles under neutral or acidic conditions (B).

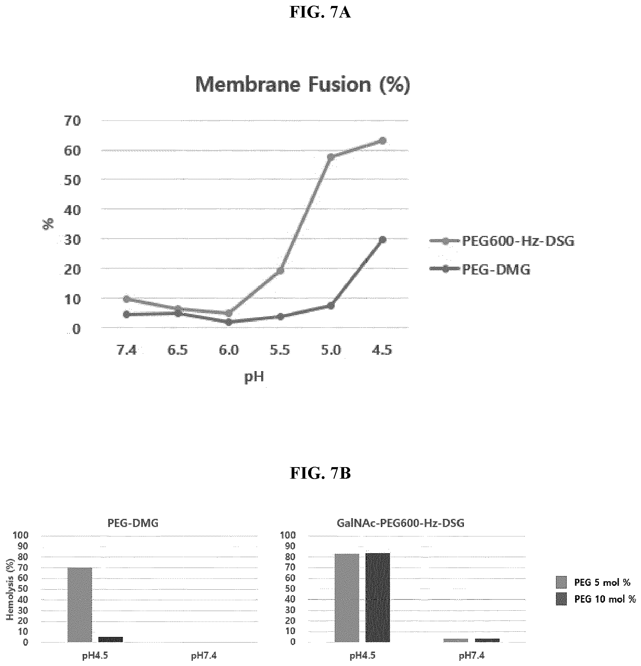

FIG. 7 shows data of the strength of fusion at the corresponding pH conditions between the liposomal membrane and lipid nanoparticles containing 10 mol% of PEG600-Hz-DSG or 1.5 mol% of PEG-DMG and a fluorescent dye according to an embodiment of the present invention (A) and data of hemolysis of lipid nanoparticles containing 5 or 10 mol% of GalNAc-PEG600-Hz-DSG or PEG-DMG under pH 4.5 and pH 7.4 conditions (B).

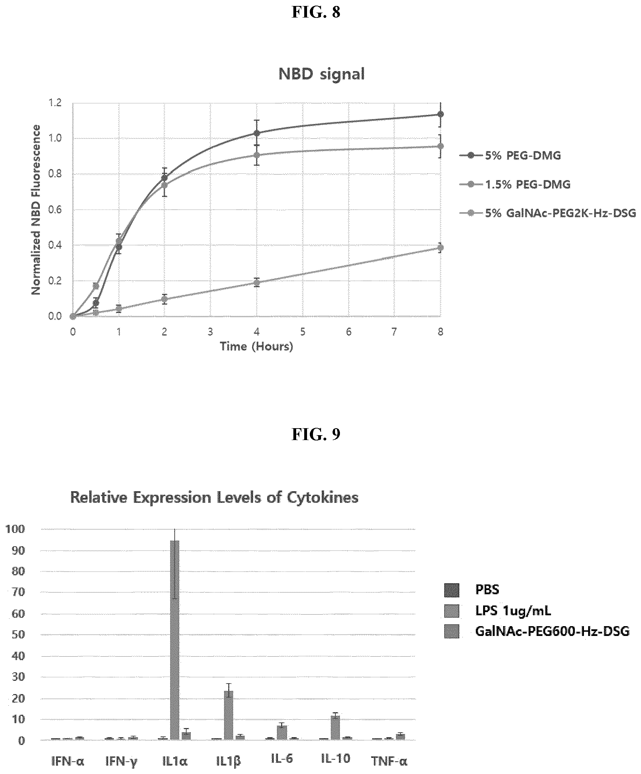

FIG. 8 shows data of change over time in fluorescence intensity contained in lipid nanoparticles when immersing, in rat plasma, lipid nanoparticles containing 1.5 mol% and 5 mol% of PEG-DMG and lipid nanoparticles containing 5 mol% of GalNAc-PEG2K-Hz-DSG based on the total content of lipids using FRET fluorescent dyes according to an embodiment of the present invention.

FIG. 9 shows data of relative amounts of produced cytokines, measured by q-PCR, when mixing lipid nanoparticles containing 10 mol% of GalNAc-PEG600-Hz-DSG with peripheral blood mononuclear cells (PBMC) isolated from rats.

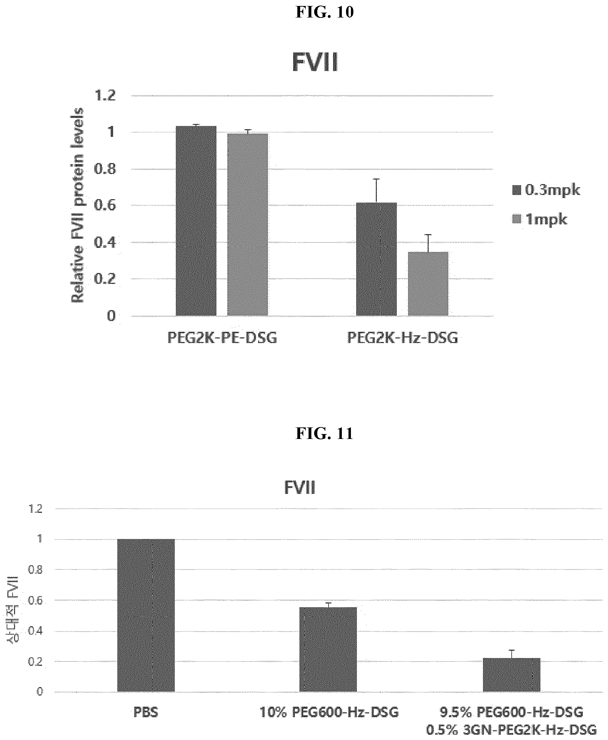

FIG. 10 shows data of the content of Factor7 in serum of mice administered with the lipid nanoparticles containing PEG2K-PE-DSG or PEG2K-Hz-DSG according to an embodiment of the present invention.

FIG. 11 shows data of the content of Factor7 in serum of mice administered with the lipid nanoparticles containing 10 mol% of PEG600-Hz-DSG or a mixed lipids of 9.5 mol% of PEG600-Hz-DSG and 0.5 mol% of GalNAc-PEG2K-Hz-DSG according to an embodiment of the present invention.

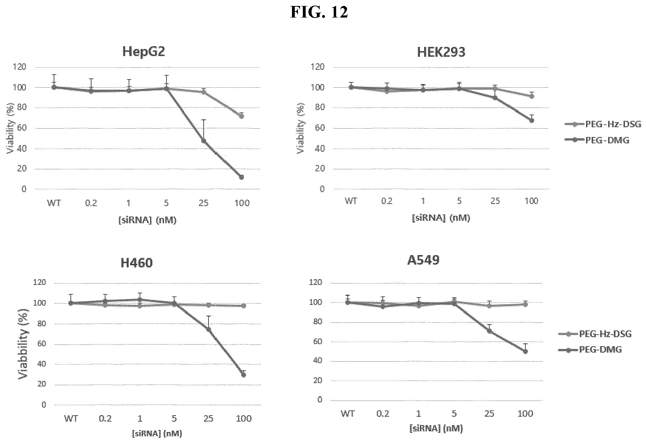

FIG. 12 shows data of relative viability of each cell line treated with lipid nanoparticles containing PEG-DMG or PEG-Hz-DSG according to an embodiment of the present invention.

### [72] [Best Mode]

Unless defined otherwise, all technical and scientific terms used herein have the same meanings as appreciated by those skilled in the field to which the present invention pertains. In general, the nomenclature used herein is well-known in the art and is ordinarily used.

The present invention is based on the finding that, when lipid nanoparticles containing an ionizable lipid and a polyethylene glycol derivative moiety-degradable functional group-lipid as components are produced and used as a therapeutic agent, they can minimize side effects *in vivo* caused by the components of the nanoparticles, effectively transfer the nanoparticles to target cells, and are suitable for effectively delivering pharmacological substances such as nucleic acids from endosomes to the cytoplasm in the cells. Based thereon, the present invention has been completed.

In one aspect, the present invention is directed to a lipid nanoparticle including (a) a lipid formulations containing an ionizable lipid and a polyethylene glycol moiety (PEG moiety)-degradable functional group-lipid conjugate, and (b) a drug, nucleic acid, or combination thereof encapsulated in the lipid formulations.

In another aspect, the present invention is directed to a method of producing the lipid nanoparticle including the steps of (a) mixing an organic solution containing lipid formulations containing an ionizable lipid and a polyethylene glycol moiety (PEG moiety)-degradable functional group-lipid conjugate, with a buffer solution containing a drug, nucleic acid, or combination thereof and adjusting pH, and (b) removing the solvent from the solution.

Hereinafter, the present invention will be described in detail.

As used herein, the terms "polyethylene glycol", "PEG", "PEG moiety", and "PEG derivative' include functional groups corresponding to polyethylene glycol and may be interchangeably used. Combinations of the elements are encompassed in all possible variations unless otherwise indicated herein or otherwise clearly contradicted by context.

The lipid nanoparticle according to the present invention includes (a) a lipid formulations containing an ionizable lipid and a polyethylene glycol moiety (PEG moiety)-degradable functional group-lipid conjugate, and (b) a drug, nucleic acid, or a combination thereof encapsulated in the lipid formulations.

In the present invention, the PEG moiety-degradable functional group-lipid conjugate may be represented by the following Formula 1.

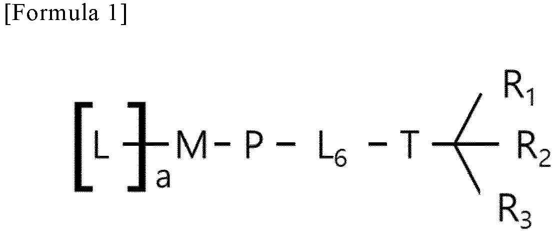

wherein
a is 0 or 1,
L is a targeting ligand,
M is H, OH, single bond, O, S, C(O), NHC(O), C(O)NH, OC(O) or C(O)O,
P is CH₂O(CH₂CH₂O)_{q}CH₂ or CH₂CH₂O(CH₂CH₂O)_{q}CH₂, in which q is an integer of 2 to 120,
L₆ is C(O)NH-N=CR₄, R₄C=N-NHC(O), NH-N=CR₄, R₄C=N-NH, C(O)O, OC(O), OC(O)O, O-N=CR₄, R₄C=N-O, S-S, S or *trans-*cyclooctene, 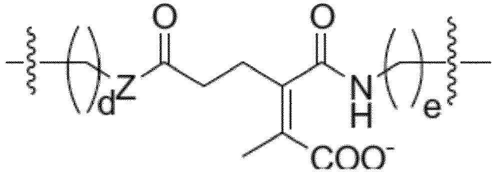
or in which, R₄ is H, C₁₋C₂₀ alkyl, C₂₋C₂₀ alkenyl, C₂₋C₂₀ alkynyl, C₃₋C₁₀ cycloalkyl, C₆₋C₂₀ aryl or a heterocycle, which is a radical containing a heteroatom selected from fluorine, oxygen, sulfur, and nitrogen, Z is NH, O or S, d is an integer of 1 to 10, and e is an integer of 1 to 10,
T is a single bond or 1,4-C₆H₄O-,
R₁ and R₃ are each independently -Y-R, R₂ is -CH₂-Y-R, in which Y is a single bond, O, S, C(O), C(O)O, OC(O), C(O)NH, or NHC(O), and R is H, C₁₀-C₂₀ alkyl, alkenyl or sterol.

In addition, a ligand may be present at one end of the PEG moiety-degradable functional group-lipid conjugate.

The ligand (L) of the PEG moiety-degradable functional group-lipid conjugate may be represented by the following Formula 2:

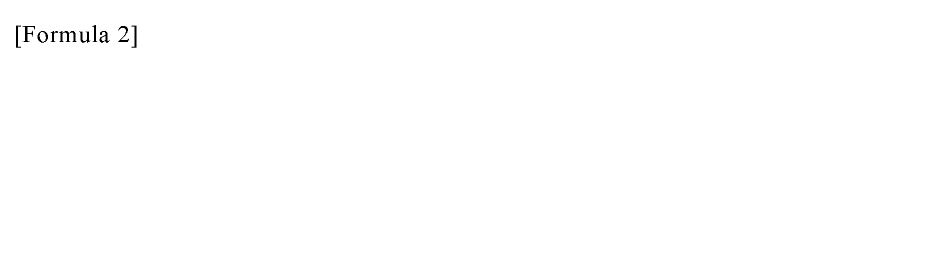

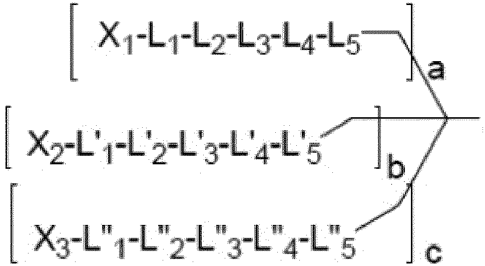

wherein
a, b and c are 0 or 1, with the proviso that at least one of a, b or c is 1,
X₁, X₂ and X₃ are targeting ligands,
L₁, L'₁ and L"₁ are a single bond, O, S, C(O), NHC(O), C(O)NH, OC(O) or C(O)O,
L₂, L'₂ and L'₂ are (CH₂)ₙ or (OCH₂CH₂)ₘ, in which n is an integer of 1 to 20 and m is an integer of 1 to 10,
L₃, L'₃ and L"₃ are a single bond, O, S, C(O), NHC(O), C(O)NH, OC(O) or C(O)O,
L₄, L'₄ and L'₄ are (CH₂)ₙ, in which n is an integer of 1 to 20, and
L₅, L'₅ and L"₅ are a single bond, O, S, C(O), NHC(O), C(O)NH, OC(O) or C(O)O.

In the present invention, X₁, X₂, and X₃ may be selected from the group consisting of N-acetyl-D-galactosamine (GalNAc), N-acetyl-D-galactose, D-galactose, N-acetyl-D-glucosamine, N-acetyl-D-glucosamine, D-glucose, D-mannose, L-fucose, carbohydrate derivatives, folate, transferrin, RGD peptides, cyclic RGD peptides, TAT peptides, R9 peptides, CADY peptides, HA2 peptides, monoclonal antibodies, antigen-binding fragments or antibody fragments, single-chain variable fragments (scFv) and aptamers. Here, the antigen-binding fragment of an antibody or the antibody fragment refers to a fragment that has an antigen-binding function and includes Fab, F(ab'), F(ab')2, Fv or the like.

In the present invention, examples of the antibody that can be used as a ligand include, but are not limited to, anti-CD3, anti-CD19, anti-CD20, anti-CD22, anti-CD33, anti-CD38, anti-CD54, anti-CD74, anti-CD138, anti-CD 166, anti-CD209, anti-cMET, anti-EGFR, anti-HER2, anti-HIV-gp120, anti-HLA-DR, anti-transferrin receptor (TfRscFv) and the like.

In the present invention, the polyethylene glycol (PEG) moiety-degradable functional group-lipid conjugate may preferably be represented by the following Formula 3 (PEG-Hz-lipid):

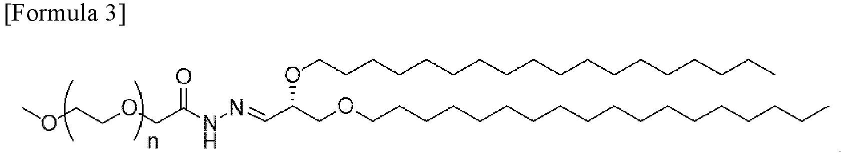

wherein n is an integer of 2 to 120.

In the present invention, the PEG moiety-degradable functional group-lipid conjugate is preferably a ligand-PEG moiety-degradable functional group-lipid conjugate (a is 1 in Formula 1), and more preferably, represented by the following Formula 4 (GalNAc-PEG-Hz-Lipid).

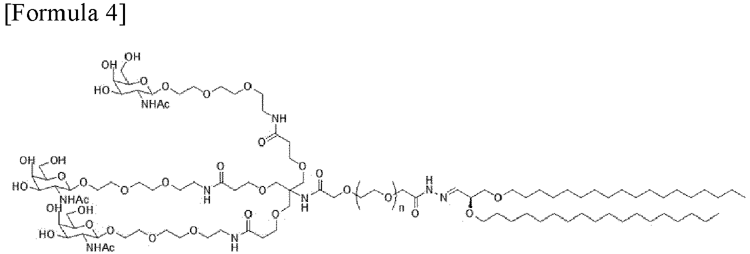

wherein n is an integer of 2 to 120.

In the present invention, the ligand-PEG moiety-degradable functional group-lipid conjugate may be a mixture of a PEG moiety-degradable functional group-lipid conjugate (a in Formula 1 is 0) and a ligand-PEG moiety-degradable functional group-lipid conjugate (a in Formula 1 is 1).

In the present invention, the molar ratio of the compound of in Formula 1 wherein a is 0 to the compound of in Formula 1 wherein a is 1 is (0.01 to 99.9):(0.01 to 99.9), preferably (50 to 99.9):(0.01 to 50), preferably, (70-99):(1-30), and most preferably, (90-95):(5-10). That is, the ligand-PEG moiety-degradable functional group-lipid conjugate may be present in an amount of 0.01 to 100 mol%, preferably 0.01 to 20 mol%, more preferably 5 to 10 mol%, of a mixture of the ligand-PEG moiety-degradable functional group-lipid conjugate and the PEG moiety-degradable functional group-lipid conjugate. In this case, the ligand-PEG moiety-degradable functional group-lipid conjugate may vary depending on the ligand bound thereto. N-acetyl-D-galactosamine (GalNAc) used in the embodiment of the present invention exhibits efficacies of the lipid nanoparticles although it is used in a small amount (5 to 10 mol%).

In the present invention, the size of the lipid nanoparticles may be 20 to 200 nm, preferably 30 to 200 nm.

The structural characteristics of the PEG moiety in the PEG moiety-degradable functional group-lipid conjugate according to the present invention are as follows.
1. The PEG moiety has a molecular weight of 100 to 5,000 and has a single or branched chemical structure. The content of the PEG moiety may be 0.5 to 50 mol%, preferably 0.5 to 40 mol%, and more preferably 0.5 to 30 mol% of the total lipid constituting the lipid nanoparticle. When the content of the PEG moiety is less than 0.5 mol%, it is difficult to obtain nanoparticles with a uniform size when producing lipid nanoparticles, and other lipid components constituting the nanoparticles are exposed to the surface, readily causing non-specific interactions. When the PEG content exceeds 50 mol%, there is a problem that the PEG moiety-degradable functional group-lipid conjugate component dose not participate in the formmation of the lipid nanoparticles but forms independent micelles instead. In addition, the molecular weight of PEG and the content of lipid nanoparticles are inversely correlated with each other. When a PEG with a high molecular weight of about 5,000 is used, the content of PEG in the lipid nanoparticles is 20 mol% or less, but when a PEG with a low molecular weight of 100 is used, the content of the PEG in the lipid nanoparticles can be increased to 50 mol%. As the molecular weight increases, it is difficult to form uniform lipid nanoparticles due to the steric effect caused by PEG, and the increase of hydrophilic PEG moiety compared to the hydrophobic lipid moiety makes the molecules have a strong tendency to form independent micelles, rather than be incorporated into the lipid nanoparticle.
2. Part or all of the polyethylene glycol (PEG) moiety-degradable functional group-lipid conjugate may be used by linking a ligand that can bind to a cell receptor at one end of the PEG moiety. The ligand includes a substance that can selectively bind to a receptor expressed on specific cells in the form of a sugar or carbohydrate including N-acetylgalactosamine, glucose, mannose, fucose, or the like, a peptide containing RGD, or the like, a small molecule such as folate, nucleic acid such as an aptamer, and a protein such as antibody or antigen-recognition partial antibody. More specifically, the ligand includes N-acetyl-D-galactosamine (GalNAc), N-acetyl-D-galactose, D-galactose , N-acetyl-D-glucosamine, N-acetyl-D-glucosamine, D-glucose, D-mannose, L-fucose, carbohydrate derivatives, folate, transferrin, RGD peptides, cyclic RGD peptides, TAT peptides, R9 peptides, CADY peptides, HA2 peptides, monoclonal antibodies, antigen-binding fragments or antibody fragments, single-chain variable fragments (scFv), aptamers and the like.
3. The opposite end of the PEG moiety is linked by a linker, which contains a chemical functional group that degrades under specific conditions or a substrate for a biological hydrolytic enzyme. For example, the linker includes -C(O)-NH-N=CR-, -RC=N-NH-C(O)-, -NH-N=CR-, - RC=N-NH-, -C(O)-O-, -O-C(O)-, -O-C(O)-O-, -O-N=CR-, -RC=N-O-, -S-S-, -S- or -trans-cyclooctene-, wherein R is H, alkyl, alkenyl, alkynyl, cycloalkyl, aryl, heterocycle, or the like.
4. The linker is linked to a hydrophobic substance such as a fatty acid-based lipid or sterol.

Among the components of lipid nanoparticles according to the present invention, the ionizable lipid is an ionizable compound linked to a lipid component and functions to encapsulate drugs (e.g., anionic drugs and/or nucleic acids) in nanoparticles through electrostatic interaction at a high efficiency. The ionizable lipids are electrically neutral at neutral pH and are positively charged at acidic pH.

In the present invention, the ionizable lipid may include at least one selected from (6Z, 9Z, 28Z, 31Z)-heptatriaconta 6,9,28,31-tetraen-19-yl 4-(dimethylamino)butanoate (DLin-MC3-DMA), [(4-hydroxybutyl)azanediyl]di(hexan-6,1-diyl)bis(2-hexyldecanoate) (ALC-0315), 8-[(2-hydroxyethyl)[6-oxo-6-(undecyloxy)hexyl]amino]-octanoic acid (SM-102), 1-linoleoyl-2-linoleyloxy-3-dimethylaminopropane (DLin-2-DMAP), 1,2-dilinoleyl carbamoyloxy-3-dimethylaminopropane (DLin-C-DAP), 1,2-dilinoleoyl-3-dimethylaminopropane (DLin-DAP), 1,2-dilinoleyloxy-N,N-dimethylaminopropane (DLin-DMA), 1,2-dilinoleyl-4-dimethylaminomethyl-[1,3]-dioxolane (DLin-K-DMA), 2,2-dilinoleyl-4-(2-dimethylaminoethyl)-[1,3]-dioxolane (DLin-KC2-DMA), 1,2-dioleoyl-3-dimethylammonium propane (DODAP), N,N-dimethyl-(2,3-dioleyloxy)propylamine (DODMA), dioctadecylamidoglycyl-spermine (DOGS), spermine cholesteryl carbamate (GL-67), bis-guanidinium-spermidine-cholesterol (BGTC), 3*β-*(N-(N',N'-dimethylaminoethane)-carbamoyl) cholesterol (DC-Chol), 1,1'-(2-(4-(2-((2-(bis(2-hydroxydecyl)amino)ethyl)(2-hydroxydecyl)amino)ethyl)piperazin-1-yl)ethyazandiyl)didodecan-2-ol (C12-200), N-t-butyl-N'-tetradecylamino-propionamidine (diC14-amidine), dimethyl dioctadecyl ammonium bromide (DDAB), N-(1,2-dimyristyl oxyprop-3-yl)-N,N-dimethyl-N-hydroxyethylammonium bromide (DMRIE), N,N-dioleyl-N,N-dimethylammonium chloride (DODAC), dioleyloxypropyl-3-dimethylhydroxyethylammonium bromide (DORIE), N-(1-(2,3-dioleyloxyl)propyl)-N-2-(sperminecarboxamid)ethyl)-N,N-dimethylammonium trifluoroacetate (DOSPA), 1,2-dioleoyl trimethylammonium propane chloride (DOTAP), N-(1-(2,3-dioleyloxy)propyl)-N,N, N-trimethylammonium chloride (DOTMA) and aminopropyl-dimethyl-bis(dodecyloxy)-propane amidium bromide (GAP-DLRIE).

Preferably, the ionizable lipid may be a compound represented by Formula 5 (DLin-MC3-DMA), Formula 6 (ALC-0315), or Formula 7 (Lipid H (SM-102)).

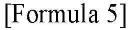

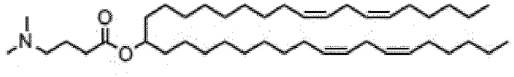

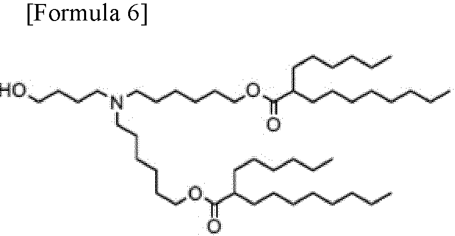

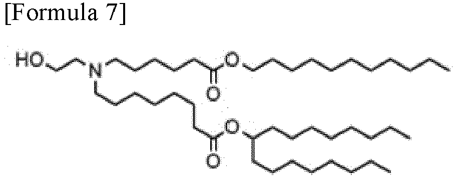

Lipid nanoparticles according to the present invention may further include sterol lipids and neutral lipids.

Among the components of lipid nanoparticles according to the present invention, the neutral lipid functions to surround and protect the core formed by the interaction of ionizable lipids and drugs within the lipid nanoparticles, and binds to the lipid bilayer of the target cell to facilitate passage of the drugs through the cell membrane and escape from endosomes during delivery of drugs to the cells. Among the components of the lipid nanoparticle according to the present invention, the neutral lipid may be used without limitation as long as it is a phospholipid or sphingolipid that can promote the fusion of lipid particles, and is preferably DOPE (dioleoylphosphatidylethanolamine), DSPC (distearoylphosphatidylcholine), POPC (palmitoyloleoylphosphatidylcholine), EPC (egg phosphatidylcholine), DOPC (dioleoylphosphatidylcholine), DPPC (dipalmitoylphosphatidylcholine), DOPG (dioleoylphosphatidylglycerol), DPPG (dipalmitoylphosphatidylglycerol), DSPE (distearoylphosphatidylethanolamine), PE (phosphatidylethanolamine), DPPE (dipalmitoylphosphatidylethanolamine), DOPE (1,2-dioleoyl-sn-glycero-3-phosphoethanolamine), POPE (1-palmitoyl-2-oleoyl-sn-glycero-3-phosphoethanolamine), POPC (1-palmitoyl-2-oleoyl-sn-glycero-3-phosphocholine), DOPS (1,2-dioleoyl-sn-glycero-3-[phospho-L-serine]), ceramide, or sphingomyelin.

Among the components of the lipid nanoparticles according to the present invention, the sterol lipid imparts morphological rigidity to lipids filling the lipid nanoparticles, is dispersed on the core and surface of the nanoparticles to improve the stability of the nanoparticles, and may be cholesterol and a cholesterol derivative including cholesteryl ester.

In the present invention, the drug may include at least one selected from the group consisting of peptides, protein drugs, protein-nucleic acid structures, and anionic biopolymer-drug conjugates.

In the present invention, the nucleic acid may be selected from the group consisting of single-stranded siRNA, double-stranded siRNA, rRNA, DNA, cDNA, plasmids, aptamers, mRNA, tRNA, lncRNA, piRNA, circRNA, saRNA, antisense oligonucleotides, shRNA, miRNA, ribozyme, PNA, and DNAzyme.

In the present invention, siRNA may target genes for metabolic diseases, including nonalcoholic steatohepatitis, such as ACACA, ANGPTL3, AOC3, MAP3K5, CCR2, CCR5, DGAT2, DPP4, FASN, HSD17B13, KHK, LGALS3, LOXL2, METAP2, MPC, NOX1, NOX4, PNPLA3, SCD, SGLT1, and SGLT2, genes for hepatitis B virus (HBV) such as HBV-S, HBV-P, and HBV-X as an example of viral diseases, TTR genes for TTR-mediated amyloidosis, ALAS1 genes for acute hepatic porphyrias, HAO1 genes for primary hyperoxaluria, PCSK9 genes for hypercholesterolemia, AT genes for hemophilia, C5 genes for complement-mediated diseases, AAT genes for Alpha-1 liver diseases, AGT genes for hypertension, AAT genes for Alpha-1 antitrypsin-deficient diseases, APOC3 genes for hypertriglyceridemia, ANGPTL3 genes for hypertriglyceridemia, LA genes for cardiovascular diseases, HSP47 genes for idiopathic pulmonary fibrosis, LDHA genes for primary hyperoxaluria, CTGF for hypertrophic and keloid scars, CTNNB 1, HSP90, KRAS, EPHA2, BCL2L12, PKN3, and HIF-2alpha genes for cancer, and alphaENaC genes for cystic fibrosis, but is not limited thereto.

In the present invention, the mRNA is mRNAs expressing antigens for vaccines, mRNAs expressing antibodies for immunotherapeutic agents, mRNAs expressing defective proteins due to genetic mutation, or the like, and mRNAs and sgRNAs expressing the Cas protein and guide RNA using CRISPR for specific DNA gene editing, and the like. Examples of antigen-expressing mRNA for vaccine development include glycoprotein mRNA of rabies virus for rabies vaccine, glycoprotein mRNA of respiratory syncytial virus vaccine, and stabilized prefusion F protein mRNA, Zika virus structural protein mRNA from Zika virus vaccine, viral antigen protein mRNA from chikungunya virus vaccine, petameric viral antigen from cytomegalovirus vaccine, gB protein mRNA, influenza vaccine HA protein mRNA, HIV immunogen protein mRNA for T cell activation of HIV vaccine, and the like. Examples of mRNA for the development of anticancer drugs or anticancer vaccines include mRNA expressing specific overexpressed protein antigens derived from patients, mRNA expressing antigens including PAP, PSA, PSCA, PSMA, and STEAP1 in prostate cancer, MUC1, CEA, Her-2neu, telomerase, survivin, and MAGE-A1 mRNA in renal cancer, Melan-A, tyrosinase, gp100, MAGE-A1, MAGE-A3, survivin mRNA, and cytokine mRNAs including IL-23, IL-36, IL-12, IL-15, GM-CSF, INF-alpha, IL-7, IL-2, and the like as vaccines or treatments for various solid cancers or blood cancers. In addition, examples of mRNA that directly expresses an antibody *in vivo* using mRNA that expresses an antibody include mRNA that expresses a chikungunya virus neutralizing monoclonal antibody, but is not limited thereto.

The lipid nanoparticle according to the present invention may be produced by (a) mixing an organic solution containing a lipid formulation containing an ionizable lipid and a polyethylene glycol moiety (PEG moiety)-degradable functional group-lipid conjugate, with a buffer solution containing a drug, nucleic acid, or combination thereof and adjusting pH, and (b) removing the solvent from the solution.

The lipid nanoparticles according to the present invention may be produced by mixing lipid components dissolved in an organic phase with oligos dissolved in an aqueous buffer solution. Because the polarity of the solvent changes as the organic phase is mixed with the aqueous solution, the lipid component contained in the organic phase spontaneously forms particles in the mixed solution. At this time, when the ionizable lipid contained in the organic phase is mixed with an acidic aqueous buffer solution containing oligos, the ionizable lipids become cationic, the cationic ionizable lipids bind to the anionic oligos by electrostatic attraction to produce nanoparticles containing oligos.

In the method for producing lipid nanoparticles according to the present invention, the mixing ratio (volume ratio) of the organic solution to the buffer solution may be 1:1 to 1:100.

### Efficiency depending on content of PEG in lipid nanoparticles

The PEG-lipid conjugate is generally used in an amount of 1 to 2 mol% to produce lipid nanoparticles. When the lipid nanoparticles are produced without the PEG-lipid, the resulting particles aggregate with each other. In order to prevent this phenomenon, a predetermined amount of PEG-lipid conjugate should be incorporated to produce lipid nanoparticles. The lipid moiety of the incorporated PEG-lipid conjugate is disposed in a hydrophobic environment that constitutes the interior of the resulting nanoparticles, and the hydrophilic PEG moiety is disposed on the surface of the particle to suppress aggregation between nanoparticles. On the other hand, nanoparticles having an excessively high PEG content can inhibit the interaction between lipid nanoparticles and cells as well due to the steric effect of PEG exposed on the particle surface. As a result, lipid nanoparticles cannot be effectively delivered into cells.

When lipid nanoparticles are produced using PEG-DMG (1,2-dimyristoyl-3-PEG-glycerol), which is a PEG-lipid conjugate commonly used in the production of lipid nanoparticles, as the PEG content increases, the cellular uptake efficiency decreases and the ability of siRNA to degrade mRNA also decreases. However, since some or all of the PEG moiety-degradable functional group-lipid conjugate according to the present invention contains, at one end of PEG, a ligand that specifically binds to a receptor expressed on specific cells, lipid nanoparticles produced using them have high cellular uptake efficiency regardless of the content of PEG moiety-degradable functional group-lipid conjugate. In other words, the inhibition of cellular uptake of lipid nanoparticles due to the steric effect of PEG can be overcome using the ligand.

### Confirmation of PEG removal from lipid nanoparticles

For lipid nanoparticles to deliver pharmocological substances such as nucleic acids into cells to induce efficacy, a two-step delivery process is required. First, lipid nanoparticles must enter the endosomes of cells through the cellular uptake process. In the second step, it is essential to effectively deliver the active ingredient from the endosome to the cytoplasm. It is widely known that lipid nanoparticles are encapsulated in the endosomes of cells through endocytosis and that the lipid component of lipid nanoparticles can fuse with the lipid membrane constituting the endosome and simultaneously deliver active ingredients into the cytoplasm. However, when the surface of the lipid nanoparticle is surrounded by PEG, the interaction with the endosomal lipid membrane is inhibited and delivery to the cytoplasm is thus difficult. Therefore, PEG on the surface of lipid nanoparticles inhibits interaction with cells, thus reducing the cellular uptake efficiency of lipid nanoparticles, and interfering with the interaction between nanoparticles successfully encapsulated in endosomes and the endosomal lipid membrane. Therefore, the PEG doubly inhibits the efficiency of the particles.

Therefore, it is advantageous to remove PEG from lipid nanoparticles in the endosome for effective interaction between the endosomal lipid membrane and lipid nanoparticles. However, nanoparticles containing commonly used PEG-lipid conjugates such as PEG-DMG do not have a way to remove PEG. Therefore, PEG remains on the particle surface in the endosomes. On the other hand, the PEG moiety-degradable functional group-lipid conjugate described in the present invention contains a chemical functional group between the PEG and the lipid that is hydrolyzed under acidic conditions. For example, the PEG moiety-degradable functional group-lipid conjugate shown in Formula 3 has a molecular structure in which two strands of lipids are linked to the PEG through a hydrazone functional group (-C=NNH-). The hydrazone functional group is stable at neutral pH, but is hydrolyzed under acidic conditions. When changes in the size of lipid nanoparticles produced using the PEG moiety-degradable functional group-lipid conjugate under acidic conditions according to the present invention are observed over time, PEG is removed from the surface of the particle as the hydrazone functional group is hydrolyzed, leading to the increase of the particle size due to continuous fusion of the lipid components constituting the particles. In addition, it can be seen that the fusion between particles continues over time and the size gradually increases. This is a phenomenon in which the hydrazone functional group is successfully hydrolyzed under acidic conditions, triggering fusion between particles, and this also facilitates fusion between the endosomal lipid membrane and nanoparticles.

### Induction of hemolysis of lipid nanoparticles

A well-known method for evaluating the interaction between lipid nanoparticles and endosomal lipid membranes is to measure the degree of destruction, that is, hemolysis, of red blood cells, when bringing nanoparticles in contact with the red blood cells. Here, the red blood cell membrane is considered a substitute for the lipid membrane of endosomes. The frequent occurrence of hemolysis indicates that lipid nanoparticles are fused more efficiently with the endosomal lipid membrane.

The ionizable lipid, which is a component of lipid nanoparticles, is known to play a key role in interaction with the endosomal lipid membrane. Ionizable lipids do not have a charge at neutral pH, but generally contain an amine group so that they are positively charged in the acidic environment of the endosome. The optimal pKa of the amine group is known to be between 5 and 7. Therefore, to determine the extent to which lipid nanoparticles fuse with the endosomal lipid membrane, red blood cells are mixed with nanoparticles under pH conditions lower than the pKa of the amine group and the hemolysis is measured. The ionizable lipid used in this example is DLin-MC3-DMA. The pKa of the amine group of this substance is about 6.5 and the amine group has a positive charge at a pH of 4. The positive charge can be easily fused with the negative charge of the lipid membrane of the endosome through electrostatic attraction. However, when PEG is disposed on the surface of the nanoparticle, it exhibits a shielding effect of reducing the electrostatic attraction and an effect of preventing direct contact between the surface of the nanoparticle and the lipid membrane of the endosome.

Therefore, the hemolytic reaction of lipid nanoparticles containing general PEG-DMG, which does not contain a degradable functional group, is suppressed as the PEG content increases. On the other hand, since the PEG of the PEG moiety-degradable functional group-lipid conjugate is removed through hydrolysis under acidic conditions, lipid nanoparticles containing the PEG moiety-degradable functional group-lipid conjugate as a component effectively induces a hemolytic reaction, regardless of the content of the functional group-lipid conjugate.

### Relationship between the length of lipids constituting the PEG moiety-degradable functional group-lipid conjugate and the structural stability of lipid nanoparticles

The PEG surrounding the surface of lipid nanoparticles is necessary in the lipid nanoparticle production process, but has a disadvantage in terms of pharmacological efficacy. Therefore, the lipid linked to PEG is generally myristate having 14 carbon atoms, to facilitate spontaneous removal of PEG-lipid from lipid nanoparticles while the lipid nanoparticles circulate in the bloodstream *in vivo.* This is because, as the number of carbon atoms in the lipid portion decreases, interaction with other lipid components constituting the lipid nanoparticles decreases, and the PEG-lipid conjugate is relatively easily separated and removed from the lipid nanoparticle *in vivo.* As a result, lipid nanoparticles produced using substances with relatively long fatty acids, such as palmitate (16 carbon atoms) and stearate (18 carbon atoms) cannot be easily incorporated in cell endosomes and thus the efficacy thereof is reduced since they cannot remove PEG smoothly during circulation through the bloodstream.

However, this method of removing PEG during the circulation process is very detrimental in terms of the structural stability of lipid nanoparticles. This is because when PEG-lipids begin to be separated and be removed from lipid nanoparticles, other lipid components constituting the lipid nanoparticles also begin to be lost due to the decrease in the stability of the particles. Therefore, although it is essential to remove PEG for lipid nanoparticles to have appropriate efficiency, lipid nanoparticles produced in such a way using PEG-lipid with a short lipid length have poor particle stability.

On the other hand, the PEG moiety-degradable functional group-lipid conjugate disclosed herein contains a degradable functional group that removes PEG under acidic conditions and thus can be used to form a relatively long hydrocarbon, for example, a lipid having 18 carbon atoms. Since the PEG moiety-degradable functional group-lipid conjugate remains as a component of the nanoparticle until the nanoparticles enter the endosomes of acidic conditions where the degradable functional group can be degraded, it maintains the stability of the particles during circulation through the bloodstream.

### Induction of immune responses by lipid nanoparticles

Ionizable lipids constituting lipid nanoparticles are known to stimulate immune cells and trigger an immune response. Increasing the PEG content of lipid nanoparticles can suppress direct interaction between immune cells and lipid nanoparticles, thus minimizing immune side effects caused by lipid components. However, it is difficult to increase the PEG content beyond the required minimum amount because increasing the PEG content inevitably may seriously inhibit the uptake efficiency of nanoparticles by cells and the escape of endosomes within cells.

However, the lipid nanoparticle containing the PEG moiety-degradable functional group-lipid conjugate of the present invention can increase cellular uptake efficiency using a ligand and has a degradable functional group that can remove PEG in endosomes. Therefore, when the PEG moiety-degradable functional group-lipid conjugate presented in the present invention is used, the induction of an immune response can be inhibited without decreasing the efficacy of the produced lipid nanoparticles even if the PEG content is increased. Therefore, when lipid nanoparticles using the PEG moiety-degradable functional group-lipid conjugate are mixed with peripheral blood mononuclear cells (PBMC) and the extent to which the lipid nanoparticles induce cytokines is measured, the cytokine induction level is similar to that of a PBS buffer-treated group used as a negative control.

Hereinafter, the present invention will be described in more detail with reference to examples. However, it will be obvious to those skilled in the art that these examples are provided only for illustration of the present invention and should not be construed as limiting the scope of the present invention.

### [Example]

### Example 1: Synthesis of polyethylene glycol (PEG) moiety-degradable functional group-lipid conjugate

### Example 1-1: 2-(2-(2-azidoethoxy)ethoxy)ethanol

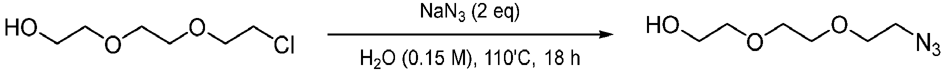

Sodium azide (1.54 g, 23.72 mmol) was added to an aqueous solution (40 mL) of 2-(2-(2-azidoethoxy)ethoxy)ethanol (2.00 g, 11.86 mmol) and stirred at 90°C for 18 hours. The reaction product was extracted three times with dichloromethane and dried over magnesium sulfate under reduced pressure to obtain the compound (2.22 g) with a yield of 98.5%.

¹H NMR (400 MHz, CDCl₃) δ 3.75-3.70 (m, 2H), 3.69-3.65 (m, 6H), 3.63-3.59 (m, 2H), 3.42-3.36 (m, 2H), 2.31 (s, 1H).

### Example 1-2: 2-methyl-3,4,6-tri-O-acetyl-1,2-deoxy-alpha-D-galactopyrano[2,1,d]-2-oxazoline

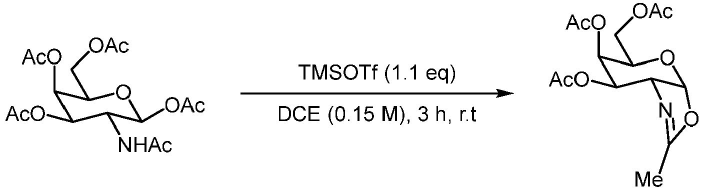

Trimethylsilyl trifluoromethanesulfonate (0.2 mL, 1.13 mmol) was added to a solution of peracetylated galactosamine (400 mg, 1.03 mmol) in DCE (22 mL) at room temperature, followed by stirring at 50°C for 4 hours. The reaction temperature was lowered to room temperature and triethylamine was slowly added thereto, followed by stirring for 15 minutes. After the reaction was completed, the organic solvent layer extracted in water and CH₂Cl₂ was dried over magnesium sulfate, filtered, and distilled under reduced pressure. The mixture obtained by distillation under reduced pressure was separated by column chromatography (CH₂Cl₂:MeOH = 40:1) to obtain the desired compound (260 mg, 0.77 mmol) with a yield of 78%.

¹H NMR (400 MHz, CDC1₃) δ 5.99 (d, *J* = 6.8 Hz, 1H), 5.46 (t, *J* = 3.0 Hz, 1H), 4.90 (dd, *J* = 7.5, 3.3 Hz, 1H), 4.26-4.08 (m, 3H), 3.99 (m, 1H), 2.12 (s, 3H), 2.07 (s, 3H), 2.07 (s, 3H), 2.05 (d, *J* = 1.2 Hz, 3H).

### Example 1-3: 5-acetamino-2-(acetoxymethyl)-6-(2-(2-(2-azidoethoxy)ethoxy)ethoxy)tetrahydro-2H-pyran-3,4-diyl diacetate

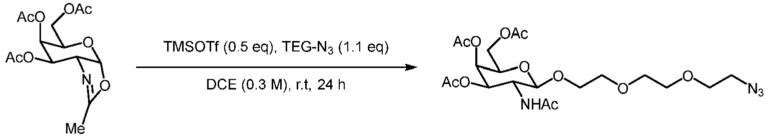

A 4 Å molecular sieve (190 mg) was added to a mixed solution (390 mg, 1.18 mmol) of 2-methyl-3,4,6-tri-*O*-acetyl-1,2-deoxy-alpha-D-galactopyrano[2,1,*d*]-2-oxazoline in DCE (7.2 mL) and 2-(2-(2-aminoethoxy)ethoxy)ethanol (228 mg, 1.30 mmol), followed by stirring for 5 minutes. Trimethylsilyl trifluoromethanesulfonate (104 µL, 0.59 mmol) was added to the resulting mixture at room temperature, followed by stirring for 16 hours. After the reaction was completed, the reaction product was filtered to remove the 4 Å molecular sieve and CH₂Cl₂ and water were added. The organic layer was separated, dried over magnesium sulfate, filtered, and distilled under reduced pressure. The mixture thus obtained was separated by column chromatography (EA:MeOH = 9:1) to obtain the desired compound (460 mg, 0.91 mmol) with a yield of 77%.

¹H NMR (400 MHz, CDCl₃): δ 6.14 (d, *J* = 9.3 Hz, 1H), 5.32 (dd, *J* = 3.4, 1.1 Hz, 1H), 5.05 (dd, *J* = 11.2, 3.4 Hz, 1H), 4.78 (d, *J* = 8.6 Hz, 1H), 4.22 (dt, *J* = 11.2, 8.9 Hz, 1H), 4.19-4.10 (m, 2H), 3.93-3.83 (m, 3H), 3.76-3.65 (m, 4H), 3.63 (dd, *J* = 7.1, 3.2 Hz, 4H), 3.51-3.39 (m, 2H), 2.15 (s, 3H), 2.04 (s, 3H), 1.99 (s, 3H), 1.98 (s, 3H).

### Example 1-4: 2-(2-(2-aminoethoxy)ethoxy)ethyl 2-acetamino-3,4,6-tri-O-acetyl-2-deoxy-α-D-galactopyranoside

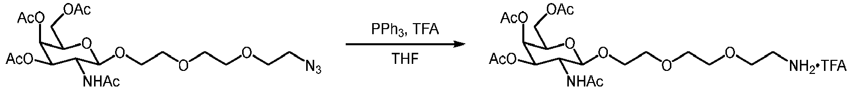

PPh₃ (150 mg, 0.571 mmol) was added to a solution of 5-acetamino-2-(acetoxymethyl)-6-(2-(2-(2-azidoethoxy)ethoxy)ethoxy)tetrahydro-2H-pyran-3,4-diyl diacetate (240 mg, 0.476 mmol) in THF (10 mL) at room temperature, followed by stirring for 48 hours. H₂O (26 µL, 1.427 mmol) was added to the reaction product, followed by stirring for 24 hours. Then, toluene (10 mL) and trifluoroacetic acid (73 µL, 0.951 mmol) were sequentially added to produce a salt. After adding water and CH₂Cl₂, the aqueous layer extracted was distilled under reduced pressure to obtain the desired compound (270 mg, 0.91 mmol) with a yield of 98%.

¹H NMR (400 MHz, CD₃OD): δ 5.35 (d, *J* = 3.2 Hz, 1H), 5.05 (dd, *J* = 11.2, 3.2 Hz, 1H), 4.58 (d, *J* = 8.4 Hz, 1H), 4.16-3.96 (m, 4H), 3.64-3.74 (m, 10H), 3.24 (m, 2H), 2.14 (s, 3H), 2.03 (s, 3H), 1.95 (s, 3H), 1.94 (s, 3H).

### Example 1-5: Tris{[2-(tert-butoxycarbonyl)ethoxy]methyl}methylamine

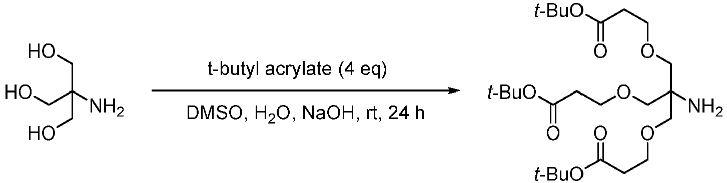

5.0 M sodium hydroxide (0.2 mL) was added to a Tris (1.21 g, 10.00 mmol) solution in DMSO (2.0 mL) at 15°C, followed by stirring for 5 minutes. *t*-Butyl acrylate (5.0 mL, 34 mmol) was added slowly dropwise thereto. Then, water (0.2 mL) was added to the reaction product, followed by stirring under argon at room temperature for 24 hours. After the reaction was completed, the mixture obtained by distillation under reduced pressure was separated by column chromatography (EA:Hex = 2:1) to obtain the desired compound (2.5 g, 5.00 mmol) with a yield of 50%.

¹H NMR (CDCl₃, 400 MHz): δ 3.65 (t, *J* = 6.0 Hz, 6H), 3.32 (s, 6H), 2.46 (t, *J* = 6.0 Hz, 6H), 1.45 (s, 27H).

### Example 1-6: (R)-((2,3-bis(octadecyloxy)propoxy)methyl)benzene

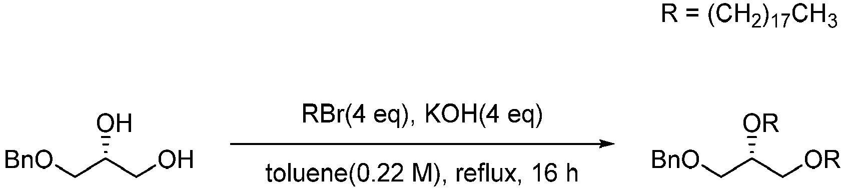

1-Bromooctadecane (732 mg, 2.20 mmol) and potassium hydroxide (123 mg) were added to a solution of (R)-3-benzyloxy-1,2-propanediol (106 mg, 0.55 mmol) in toluene (2.5 mL) at room temperature, followed by refluxing at 110°C for 24 hours. After the reaction was completed, CH₂Cl₂ and water were added. The organic solvent layer extracted was dried over magnesium sulfate, filtered, and distilled under reduced pressure. The mixture thus obtained was separated by column chromatography (Hex: EA = 10: 1) to obtain the desired compound (300 mg, 0.44 mmol) with a yield of 80%.

¹H NMR (400 MHz, CDCl₃) δ 7.32-7.21 (m, 5H), 4.55 (s, 2H), 3.64-3.48 (m, 7H), 3.43 (t, *J* = 6.7 Hz, 2H), 1.61-1.51 (m, 4H), 1.39-1.21 (m, 60H), 0.88 (t, *J* = 6.8 Hz, 6H).

### Example 1-7: (S)-2,3-bis(octadecyloxylpropan-1-ol

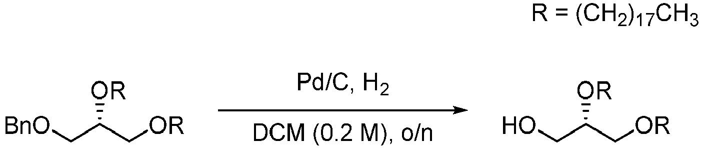

(R)-((2,3-bis(octadecyloxy)propoxy)methyl)benzene (50 mg, 0.07 mmol) was dissolved in CH₂Cl₂ (4 mL) and Pd/C (10 mg) was added dropwise thereto. The mixture was stirred at room temperature under hydrogen for 20 hours. After the reaction was completed, the mixture was filtered with ethyl acetate using Celite 545 and then distilled under reduced pressure to obtain the desired compound (40 mg, 0.07 mmol) with a yield of 92%.

¹HNMR (CDCl₃) δ 3.75-3.37 (m, 9H), 2.17 (m, 1H), 1.55 (m, 4H), 1.38-1.21 (m, 60H), 0.88 (t, *J* = 6.8 Hz, 6H).

### Example 1-8: (R)-2,3-bis(octadecyloxy)propanal

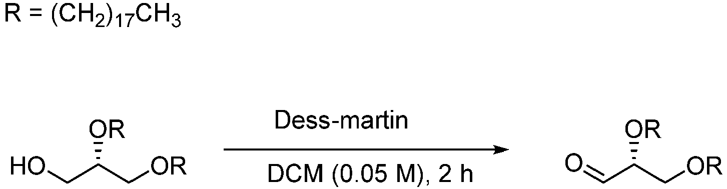

A solution of Dess-Martin periodinane (88 mg, 0.206 mmol) in CH₂Cl₂ (3.7 mL) was added dropwise slowly to (*S*)-2,3-bis(octadecyloxy)propan-1-ol (110 mg, 0.184 mmol) in CH₂Cl₂ (1.3 mL) at 0°C, followed by stirring at room temperature for 2 hours. After the reaction was completed, CH₂Cl₂, water, and aq. NaHCO₃ were added. The organic solvent layer extracted was washed with thiosulfate solution, dried over magnesium sulfate, filtered and distilled under reduced pressure. The mixture thus obtained was separated by column chromatography (Hex:EA = 20:1) to obtain the desired compound (83 mg, 0.15 mmol) with a yield of 80%.

¹H NMR (CDCl₃) δ 3.81-3.37 (m, 9H), 1.65-1.51 (m, 4H), 1.38-1.21 (m, 60H), 0.88 (t, *J* = 6.8 Hz, 6H).

### Example 1-9: carboxymethyl-PEG600-Cbz-hydrazide (Mₙ of PEG = 600)

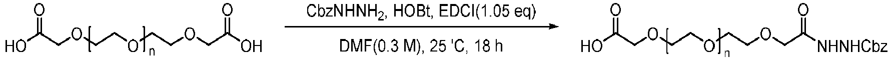

Poly(ethylene glycol)biscarboxymethyl)ether (Mₙ = 600) (1 g, 1.72 mmol) was dissolved in DMF (5.8 mL), and then CbzNHNH₂ (300 mg, 1.81 mmol), hydroxybenzotriazole (244 mg, 1.81 mmol) and 1-ethyl-3-(3-dimethylaminopropyl)carbodiimide (320 µL, 1.81 mmol) were added dropwise at 0°C to the mixture solution. The mixture was stirred at room temperature under argon for 18 hours. After the reaction was completed, CH₂Cl₂, water, and aq. NaHCO₃ were added. The separated aqueous layer was acidified with H₃PO₄ and then extracted with CH₂Cl₂. Then the organic layer was distilled under reduced pressure to obtain a product with a yield of 40%.

¹HNMR (400 MHz, CD₃OD) δ 7.39-7.30 (m, 5H), 5.15 (s, 2H), 4.13 (s, 2H), 4.10 (s, 2H), 3.73-3.60 (m, 70H).

### Example 1-10: tris{[2-(t-butoxycarbonyl)ethoxy]methyl}methylamido- PEG600-Cbz-hydrazide (Mₙ of PEG = 600)

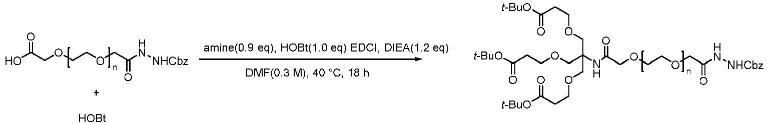

Carboxymethyl-PEG600-Cbz-hydrazide (870 mg, 1.16 mmol), and tris{[2-(t-butoxycarbonyl)ethoxy]methyl}methylamine (526 mg, 1.04 mmol) were dissolved in DMF (4 mL), and then hydroxybenzotriazole (157 mg, 1.16 mmol), 1-ethyl-3-(3-dimethylaminopropyl)carbodiimide (248 µL, 1.40 mmol), and diisopropylethylamine (243 µL, 1.40 mmol) were added dropwise thereto at 0°C. The mixture was stirred at room temperature under argon for 18 hours. After the reaction was completed, CH₂Cl₂, water, and H₃PO₄ were added. The organic solvent layer extracted was washed with aq. NaHCO₃. The organic solvent layer was dried over magnesium sulfate, filtered, and distilled under reduced pressure. The mixture thus obtained was separated by column chromatography (CH₂Cl₂ : MeOH = 15 : 1) to obtain the desired compound with a yield of 40%.

¹H NMR (400 MHz; CD₃OD) δ 7.38-7.31 (m, 5H), 5.16 (s, 2H), 4.30-4.13 (m, 3H), 4.13 (s, 2H), 3.90 (s, 2H), 3.77-3.60 (m, 70H), 2.46 (t, *J* = 6.0 Hz, 6H), 1.46 (s, 27H).

### Example 1-11: tris{[2-(carboxymethyl)ethoxy]methyt}methylamido- PEG600-Cbz-hydrazide (Mₙ of PEG = 600)

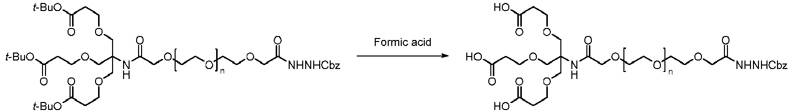

Tris{[2-(*t*-butoxycarbonyl)ethoxy]methyl}methylamido-PEG600-Cbz-hydrazide (570 mg, 0.46 mmol) was dissolved in 85% formic acid (4.0 mL), followed by stirring at room temperature for 18 hours. After the reaction was completed, the reaction mixture was distilled under reduced pressure at 50°C to obtain the desired compound with a 99% yield.

¹H NMR (CD₃OD) δ 7.38-7.30 (m, 5H), 5.15 (s, 2H), 4.30-4.17 (m, 3H), 4.13 (s, 2H), 3.90 (s, 2H), 3.78-3.63 (m, 70H), 2.53 (t, *J=* 6.0 Hz, 6H).

### Example 1-12: tri-(peracetyl-GalNAc)-PEG600-Cbz hydrazide

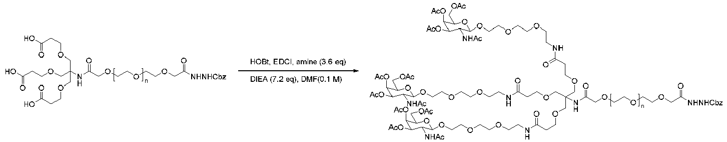

Tris{[2-(carboxymethyl)ethoxy]methyl}methylamido-PEG600-Cbz-hydrazide (0.5 mmole) and 2-(2-(2-aminoethoxy)ethoxy)ethyl 2-acetamino-3,4,6-tri-*O*-acetyl-2-deoxy-α-D-galactopyranoside (1.58 mmole) were dissolved in DMF (4.4 mL), and then hydroxybenzotriazole (214 mg, 1.58 mmol), 1-ethyl-3-(3-dimethylaminopropyl)carbodiimide (280 µL, 1.58 mmol), and diisopropylethylamine (550 µL, 3.16 mmol) were added dropwise thereto at 0°C. The mixture was stirred at room temperature under argon for 18 hours. Then, diisopropylethylamine (550 µL, 3.16 mmol) was further added dropwise at room temperature under argon, followed by stirring at room temperature for 24 hours. After the reaction was completed, CH₂Cl₂, water, and H₃PO₄ were added. The organic solvent layer was separated and washed with with aq. NaHCO₃, dried over magnesium sulfate, filtered, and distilled under reduced pressure. The mixture thus obtained was separated by column chromatography (CH₂Cl₂:MeOH = 10:1) to obtain the desired compound with a yield of 23%.

¹H NMR (400 MHz, CD₃OD) δ 7.40-7.31 (m, 5H), 5.34 (d, *J* = 3.0 Hz, 3H), 5.07 (dd, *J* = 11.2, 3.3 Hz, 3H), 5.16 (s, 2H), 4.64 (t, *J* = 8.5 Hz, 3H), 4.16-4.02 (m, 16H), 3.95-3.91 (m, 6H), 3.77-3.61 (m, 106H), 3.55 (t, *J* = 5.6 Hz, 6H), 3.38 (t, *J* = 5.6 Hz, 6H), 2.45 (t, *J* = 6.0 Hz, 6H), 2.14 (s, 9H), 2.03 (s, 9H), 1.95 (s, 9H), 1.93 (s, 9H).

### Example 1-13: tri-(peracetyl-GalNAc)-PEG600-hydrazide

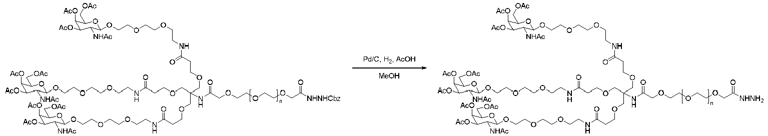

Tri-(peracetyl-GalNAc)-PEG600-Cbz hydrazide (373 mg, 0.16 mmol) was dissolved in MeOH (2 mL), and then Pd/C (13 mg) and acetic acid (28 µL, 0.48 mmol) were added dropwise thereto. The mixture was stirred at room temperature under hydrogen for 20 hours. After the reaction was completed, the mixture was filtered through Celite 545 with methanol and then distilled under reduced pressure to obtain the desired compound (120 mg, 0.05 mmol) with a yield of 99%.

¹H NMR (400 MHz, CD₃OD) δ 1.94 (s, 9H), 5.34 (d, *J* = 3.0 Hz, 3H), 5.07 (dd, *J* = 11.2, 3.4 Hz, 3H), 4.64 (d, *J* = 8.5 Hz, 3H), 4.17-3.91 (m, 16H), 3.77-3.61 (m, 106H), 3.56 (t, *J* = 5.5 Hz, 6H), 3.39 (t, *J* = 5.5 Hz, 6H), 2.46 (t, *J* = 5.9 Hz, 6H), 2.15 (s, 9H), 2.03 (s, 9H), 1.95 (s, 9H), 1.94 (s, 9H).

### Example 1-14: tri-(peracetyl-GalNac)-PEG600-(dioctadecyloxy-propylidene)-acylhydrazone

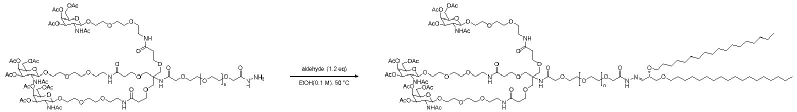

Tri-(peracetyl-GalNAc)-PEG600-hydrazide (0.05 mmol) and (*R*)-2,3-bis(octadecyloxy)propanal (39 mg, 0.06 mmol) were dissolved in a mixed solution of EtOH (1 mL) and CH₂Cl₂ (0.1 mL), followed by stirring at room temperature under argon for 3 hours. After the reaction was completed, the resulting product was distilled under reduced pressure. The mixture thus obtained was separated by column chromatography (CH₂Cl₂:MeOH = 10:1) to obtain the desired compound with a yield of 26%.

¹H NMR (CDCl₃) δ 9.92 (s, 1H), 7.43 (d, *J* = 6.9 Hz, 1H), 7.19 (m, 2H), 7.03 (s, 1H), 6.6 (s, 1H), 5.32 (d, *J* = 3.0 Hz, 3H), 5.16 (dd, *J* = 11.3, 3.0 Hz, 3H), 4.78 (d, *J* = 8.5 Hz, 3H), 4.15-3.40 (m, 134H), 2.45 (t, *J* = 5.9 Hz, 6H), 2.13 (s, 9H), 2.03 (s, 9H), 1.98 (s, 9H), 1.94 (s, 9H), 1.54-1.48 (m, 4H), 1.24 (m, 60H), 0.86 (t, *J* = 6.8 Hz, 6H).

### Example 1-15: tri-(GalNAc)-PEG600--(dioctadecyloxy-propylidene)-acylhydrazone (3GalNAc-PEG600-Hz-DSG)

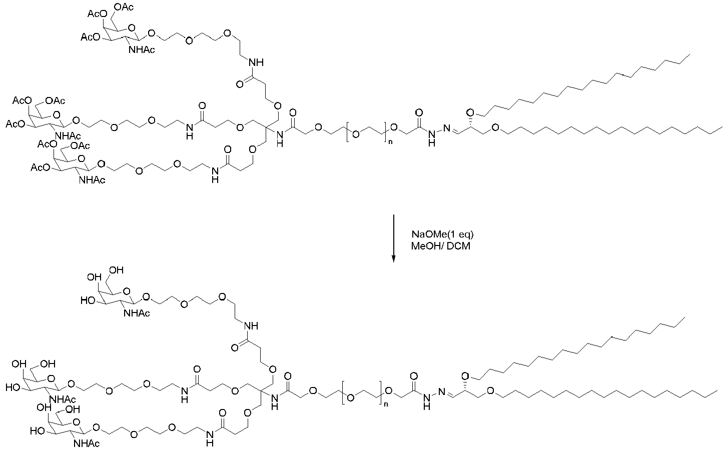

Tri-(peracetyl-GalNac)-PEG600-(dioctadecyloxy-propylidene)-acylhydrazone (0.013 mmol) was dissolved in a mixed solution of MeOH (1 mL) and CH₂Cl₂ (0.1 mL), and sodium methoxide (0.7 mg, 0.013 mmol) was added dropwise thereto. The mixture was stirred at room temperature under argon for 2 hours. After the reaction was completed, the mixture was treated with Amberlite IR-120 ion exchange resin and distilled under reduced pressure to obtain the desired compound with a yield of 26%.

¹HNMR (CDCl₃) δ 9.97(s, 1H), 8.54 (s, 1H), 7.90(m, 2H), 7.72 (m, 1H), 4.47 (m, 3H), 4.15-3.44 (m, 144H), 2.47 (t, *J* = 5.9 Hz, 6H), 2.00 (s, 9H), 1.55 (m, 4H), 1.24 (m, 60H), 0.87 (t, *J* = 6.8 Hz, 6H).

### Example 1-16: carboxymethyl-PEG2000-Cbz-hydrazide (Mₙ of PEG = 2000)

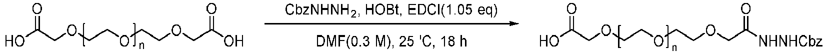

The desired compound with a yield of 40% was obtained in the same manner as in Example 1-9.

¹H NMR (400 MHz, CD₃OD) δ 7.39-7.30 (m, 5H), 5.15 (s, 2H), 4.13 (s, 2H), 4.10 (s, 2H), 3.73-3.68 (m, 190H).

### Example 1-17: tris{[2-(t-butoxycarbonyl)ethoxy]methyl}methylamido- PEG2000-Cbz-hydrazide (Mₙ of PEG = 2000)

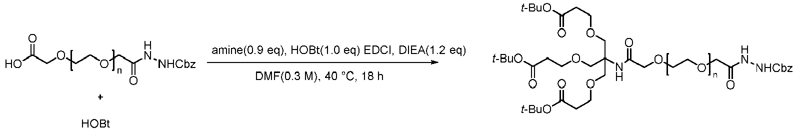

The desired compound with a yield of 30% was obtained in the same manner as in Example 1-10.

¹H NMR (400 MHz; CD₃OD) δ 7.38-7.31 (m, 5H), 5.16 (s, 2H), 4.30-4.13 (m, 3H), 4.13 (s, 2H), 3.90 (s, 2H), 3.77-3.60 (m, 190H), 2.46 (t, *J* = 6.0 Hz, 6H), 1.46 (s, 27H).

### Example 1-18: tris-{[2-(carboxymethyl)ethoxylmethyl]methy}amido- PEG2000-Cbz-hydrazide (Mₙ of PEG = 2000)

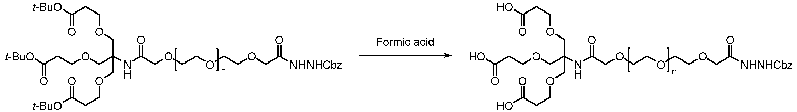

The desired compound with a yield of 99% was obtained in the same manner as in Example 1-11.

¹H NMR (CD₃OD) δ 7.38-7.30 (m, 5H), 5.15 (s, 2H), 4.12 (s, 4H), 3.78-3.96 (m, 190H), 2.53 (t, *J* = 6.0 Hz, 6H).

### Example 1-19: tri-(peracetyl-GalNAc)-PEG2K-Cbz hydrazide

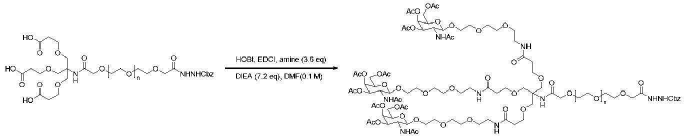

The desired compound with a yield of 30% was obtained in the same manner as in Example 1-12.

¹H NMR (400 MHz, CD₃OD) δ 7.40-7.31 (m, 5H), 5.34 (d, *J* = 3.0 Hz, 3H), 5.16 (s, 2H), 5.07 (dd, *J* = 11.2, 3.3 Hz, 3H), 4.64 (t, *J* = 8.5 Hz, 3H), 4.16-4.02 (m, 16H), 3.95-3.91 (m, 6H), 3.77-3.61 (m, 226H), 3.55 (t, *J* = 5.6 Hz, 6H), 3.38 (t, *J* = 5.6 Hz, 6H), 2.45 (t, *J* = 6.0 Hz, 6H), 2.14 (s, 9H), 2.03 (s, 9H), 1.95 (s, 9H), 1.93 (s, 9H).

### Example 1-20: tri-(peracetyl-GalNAc)-PEG2K-hydrazide

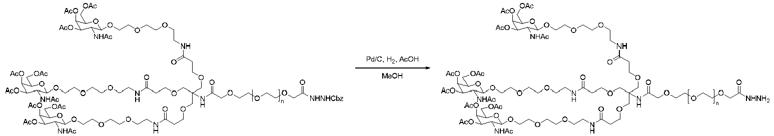

The desired compound with a yield of 99% was obtained in the same manner as in Example 1-13.

¹H NMR (400 MHz, CD₃OD) δ 5.34 (d, *J* = 3.0 Hz, 3H), 5.07 (dd, *J* = 11.2, 3.4 Hz 3H), 4.64 (d, *J* = 8.5 Hz, 3H), 4.17-3.91 (m, 16H), 3.77-3.60 (m, 226H), 3.56 (t, *J* = 5.5 Hz, 6H), 3.39 (t, *J* = 5.5 Hz, 6H), 2.46 (t, *J* = 5.9 Hz, 6H), 2.15 (s, 9H), 2.03 (s, 9H), 1.95 (s, 9H), 1.94 (s, 9H).

### Example 1-21: tri-(peracetyl-GalNac)-PEG2K-(dioctadecyloxy-propylidene)-acylhydrazone

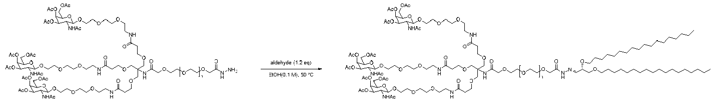

The desired compound with a yield of 37% was obtained in the same manner as in Example 1-14.

¹H NMR (CDCl₃) δ 9.95 (s, 1H), 7.43 (d, *J* = 6.9 Hz, 1H), 7.19 (m, 2H), 7.03 (s, 1H), 6.6 (s, 1H), 5.32 (d, *J* = 3.0 Hz, 3H), 5.16 (dd, *J* = 11.3, 3.0 Hz, 3H), 4.78 (d, *J* = 8.5 Hz, 3H), 4.15-3.40 (m, 226H), 2.45 (t, *J* = 5.9 Hz, 6H), 2.13 (s, 9H), 2.03 (s, 9H), 1.98 (s, 9H), 1.94 (s, 9H), 1.54-1.48 (m, 4H), 1.24 (m, 60H), 0.86 (t, *J* = 6.8 Hz, 6H).

### Example 1-22: tri-(GalNAc)-PEG2K--(dioctadecyloxy-propylidene)-acylhydrazone (3GalNAc-PEG2K-Hz-DSG)

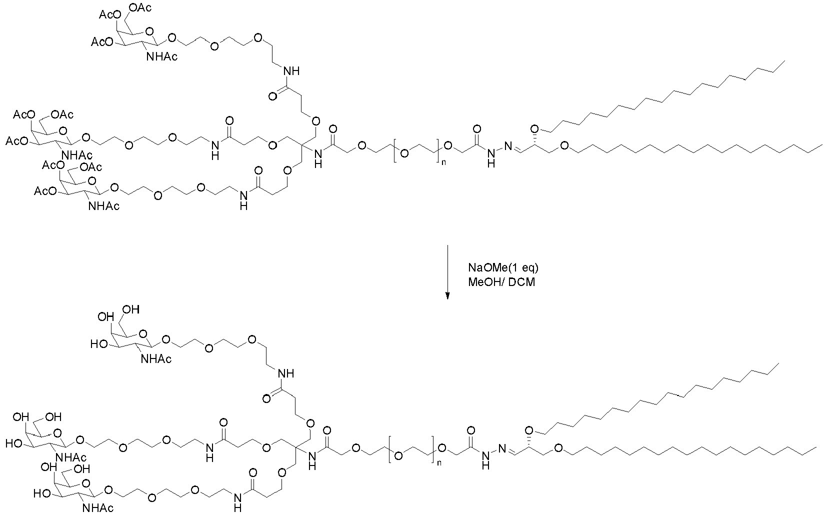

The desired compound with a yield of 26% was obtained in the same manner as in Example 1-15.

¹HNMR (CDCl₃) δ 9.93 (s, 1H), 8.54 (s, 1H), 7.90 (m, 2H), 7.72 (m, 1H), 4.47 (m, 3H), 4.15-3.44 (m, 236H), 2.47 (t, *J* = 5.9 Hz, 6H), 2.00 (s, 9H), 1.55 (m, 4H), 1.24 (m, 60H), 0.87 (t, *J* = 6.8 Hz, 6H).

### Example 1-23. methyl-PEG600-Cbz hydrazide (Mn of PEG = 600)

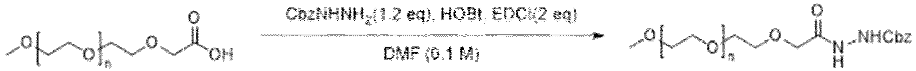

α-Methyl-ω-carboxymethyl-poly(ethylene glycol)ether (Mₙ of PEG =600) (150 mg, 0.27 mmol) was dissolved in 2.7 mL of DMF. CbzNHNH₂ (50 mg, 0.3 mmol), EDCI (58 µL, 0.33 mmol) and HOBt (44 mg, 0.33 mmol) was added thereto at 0°C, followed by stirring at room temperature for one day. After completion of the reaction, the reaction product was transferred to DCM (40 mL), washed with diluted H₃PO₄ (1 M, 40 mL) and an aqueous NaHCO₃ (1 M, 40 mL) solution, and dried over magnesium sulfate. The reaction was separated on a silica gel column with DCM/methanol (10/1, v/v) to obtain 175 mg of the compound with a yield of 91%.

¹H NMR (400 MHz, MeOH-d₄) δ 7.39-7.30 (m, 5H), 5.16 (s, 2H), 4.20 (s, 2H), 3.68-3.52 (m, 50H). 3.37 (s, 3H)

### Example 1-24: methyl-PEG600-hydrazide (Mn of PEG = 600)

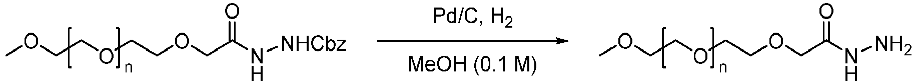

methyl-PEG600-Cbz hydrazide (175 mg, 0.25 mmol) was dissolved in methanol (2.8 mL), Pd/C (18 mg, 10 w/w%) was added thereto, and the mixture was stirred for 20 hours under hydrogen. After completion of the reaction, the mixture was filtered through Celite 545 to obtain 147 mg of the compound with a yield of 95%.

¹H NMR (400 MHz, CDC1₃) δ 4.14-4.01 (m, 2H), 3.61 (m, 50 H), 3.34 (s, 3H)

### Example 1-25: methyl-PEG600-(dioctadecyloxy-propylidene)-acylhydrazone (mPEG600-Hz-DSG)

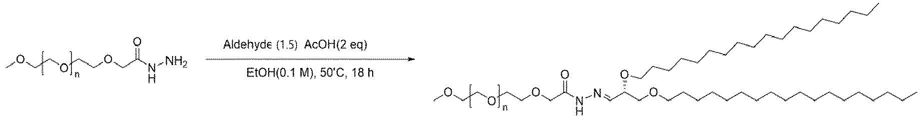

The resulting product of Example 1-24 (79 mg, 0.127 mmol) was dissolved in 1.3 mL of ethanol and the aldehyde (113 mg, 0.19 mmol) was added to the resulting product of Example 1-8. The mixture was stirred at 50°C for 18 hours to terminate the reaction. The reaction product was purified by column chromatography to obtain 123 mg of the compound with a yield of 85%.

¹H NMR (400 MHz, CDC1₃) δ 9.97(s, 1H), 7.38 (d, J = 7.0 Hz, 1H), 4.15 (m, 3 H), 3.65-3.40 (m, 56 H), 3.36(s, 3H), 1.52(m, 4H), 1.23 (m, 60 H), 0.86 (t, J = 6.4 Hz, 3H).

### Example 1-26: methyl-PEG2K-Cbz hydrazide (Mn of PEG = 2000)

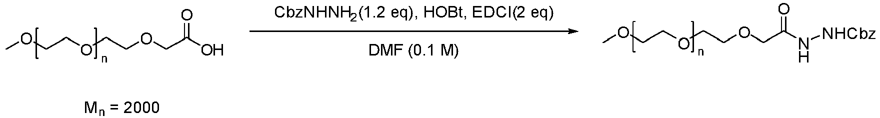

160 mg of the compound was obtained with a yield of 95% in the same manner as in Example 1-23.

¹HNMR (400 MHz, MeOH-d₄) δ 7.39-7.30 (m, 5H), 5.16 (s, 2H), 4.20 (s, 2H), 3.68-3.52 (m, 190H). 3.36 (s, 3H)

### Example 1-27: methyl-PEG2K-hydrazide (Mn of PEG = 2000) Linker Mₙ 2000

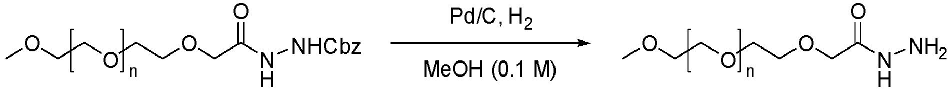

110 mg of the compound was obtained with a yield of 75% in the same manner as in Example 1-24.

¹H NMR (400 MHz, CDC1₃) δ 4.14-4.01 (m, 2H), 3.61 (m, 190 H), 3.34 (s, 3H)

### Example 1-28: methyl-PEG2K-(dioctadecyloxy-propylidene)-acylhydrazone (mPEG2K-Hz-DSG)

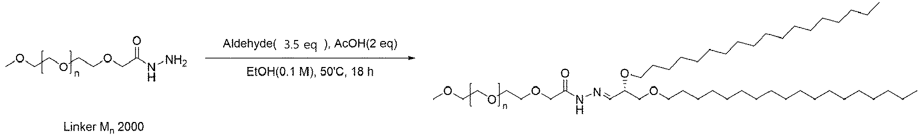

57 mg of the compound was obtained with a yield of 53% in the same manner as in Example 1-25.

¹H NMR (400 MHz, CDC1₃) δ 9.95(s, 1H), 7.36 (d, J = 7.0 Hz, 1H), 4.14 (m, 3 H), 3.60-3.40 (m, 200 H), 3.34(s, 3H), 1.51(m, 4H), 1.21 (m, 60 H), 0.84 (t, J = 6.4 Hz, 3H).

### Example 1-29: (R)-1-(4-(2,3-bis(octadecyloxy)propoxy)phenyl)ethan-1-one

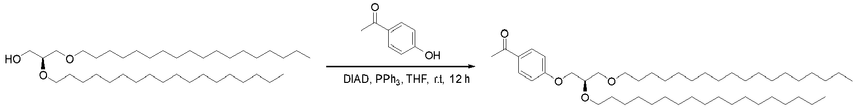

The resulting product of Example 1-7 (59.8 mg, 100 µmol) was dissolved in 2 mL of THF, and 4-hydroxyacetophenone (15.0 mg, 110 µmol), PPh₃ (39.4 mg, 150 µmol), and DIAD (30 uL, 150 µmol) were added thereto at 0°C. The mixture was stirred at room temperature for 12 hours and the reaction was terminated. The reaction product was extracted three times with dichloromethane and washed with brine. The organic layer was dried over anhydrous magnesium sulfate and dried under reduced pressure. The resulting product was purified by silica gel chromatography to obtain 55.0 mg of the compound with a yield of 77%.

¹H-NMR (400 MHz, CDC1₃) δ 7.92 (d, J = 8.8 Hz, 2H), 6.95 (d, J = 8.8 Hz, 2H), 4.19-4.05 (m, 2H), 3.79 (quin, J = 5.1 Hz, 1H), 3.64-3.57 (m, 4H), 3.46 (t, J = 6.6 Hz, 2H), 2.55 (s, 3H), 1.61-1.50 (m, 4H), 1.32-1.21(m, 60H), 0.88 (t, J = 6.8 Hz).

### Example 1-30: methyl-PEG600-(2,3-dioctadecyloxypropoxy)phenyl-acetohydrazone (mPEG600-PhHz-DSG)

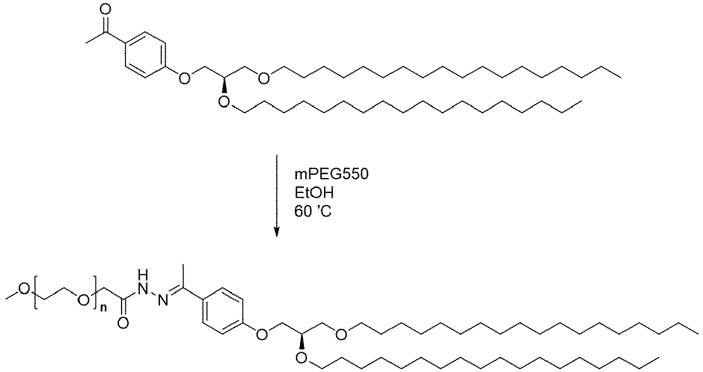

m-PEG-hydrazide (Mn = 550, Creative PEGworks, 200 mg, 364 µmol) was dissolved in ethanol (4 mL), and the resulting product of Example 1-29 (260 mg, 364 µmol) and acetic acid

(100 uL, 3.64 mmol) were added thereto. The mixture was stirred at 85°C for 16 hours. The reaction product was purified by column purification to obtain 265 mg of the product with a yield of 66%.

### Example 1-31: methyl-PEG600-(2,3-dioctadecyloxyvropoxy)carbonyl methyl ether (mPEG600-Ester-DSG)

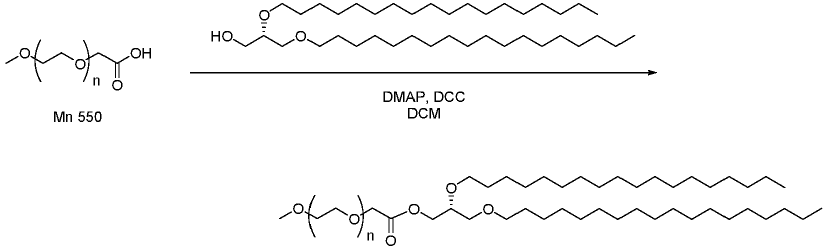

ω-(methoxypolyethylene glycol) carboxylic acid (Mₙ = 600) (100 mg, 0.156 mmol) was dissolved in DCM (30.0 mL), and (S)-2,3-bis(octadecyloxy)propan-1-ol (187 mg 0.312 mmol), 4-dimethyl aminopyridine (23 mg, 0.187 mmol) and dicyclohexylcarbodiimide (37 mg, 0.180 mmol) were added dropwise thereto. The mixture was stirred at room temperature under nitrogen for 24 hours. After the reaction was completed, the reaction product was filtered through Celite545 with CH₂Cl₂. The organic layer was washed with water, dried over magnesium sulfate, filtered, and distilled under reduced pressure. The mixture thus obtained was separated by column chromatography (DCM: MeOH = 20: 1) to obtain the desired compound (68 mg, 0.64 mmol) with a yield of 41%.

¹H NMR (500 MHz, CDC1₃) δ 4.24 (t, 2H), 3.60-3.70 (m, 33H), 3.42 (s, 2H), 1.96-1.98 (d, 3H), 1.75-1.76 (d, 3H), 1.29 (s, 60H), 0.92 (t, 6H)

### Example 2: Production of lipid nanoparticles

Two types of nanoparticles were produced to determine whether or not the PEG moiety-degradable functional group-lipid conjugate disclosed herein, that is, the PEG derivative containing a functional group that is degraded under acidic conditions, can stably form nanoparticles under acidic conditions for producing lipid nanoparticles. One contains PEG2K-Hz-DSG (Formula 3), which has a hydrazone functional group that is hydrolyzed under acidic conditions, and the other contains PEG2K-PE-DSG ((1,2-distearoyl-sn-glycero-3-phosphoethanolamine-N-[methoxy(polyethylene glycol)]-2000, Avanti), which is not hydrolyzed under acidic conditions. Two solutions were prepared to produce nanoparticles containing ionizable lipids in accordance with a conventional method. One is an organic solution containing lipid formulation, which was prepared by dissolving lipid components constituting lipid nanoparticles in ethanol. To produce lipid nanoparticles containing 1 mol% of PEG2K-Hz-DSG or PEG2K-PE-DSG, the solution was prepared with DLin-MC3-DMA, DSPC (distearoylphosphatidylcholine), cholesterol, and PEG2K-Hz-DSG or PEG2K-PE-DSG at a total concentration of 4.5 mg/mL and at the molar ratio of 50:10:39:1. In order to produce lipid nanoparticles containing PEG2K-Hz-DSG or PEG2K-PE-DSG at a ratio of 2 and 5 mol%, the corresponding PEG-lipid was used in an amount 2 to 5 times the PEG content used to produce lipid nanoparticles containing 1 mol% PEG.

Meanwhile, siRNA was prepared at a concentration of 0.046 mg/mL in a citric acid buffer solution at pH of 4. Lipid nanoparticles were produced by mixing an ethanol solution with a buffer solution at a volume ratio of 3:1 using a micro mixer chip or T-line chip, and setting the flow rate of the mixed solution to 4 mL/min. The solution that passed through the chip was further mixed with a buffer at a volume ratio of 1:1 to change the pH to 7, and then the resulting mixture was dialyzed in a pH 7.5 PBS buffer solution for 24 hours. The size of the produced lipid nanoparticles was measured using a dynamic light scattering (DLS) device. If the hydrazone of PEG2K-Hz-DSG had been hydrolyzed and decomposed into PEG and lipid (DSG) under acidic conditions to produce lipid nanoparticles, lipid components shoud have precipitaed as aggregates with a heterogeneous size, like the nanoparticles not containing the PEG moiety-degradable functional group-lipid conjugate. However, as can be seen from FIG. 2, nanoparticles containing PEG2K-Hz-DSG have uniform size, like nanoparticles containing PEG2K-PE-DSG, and as the PEG content increases, the size of nanoparticles decreases. This example shows that the PEG moiety-degradable functional group-lipid conjugate containing a functional group that is generally degraded under acidic conditions can stably form nanoparticles rather than being degraded under acidic conditions required for producing lipid nanoparticles.

### Example 3: Comparison of efficacy between lipid nanoparticles containing PEG-Hz-DSG or PEG-PE-DSG

In order to determine the efficacy of lipid nanoparticles containing PEG-Hz-DSG or PEG-PE-DSG, lipid nanoparticles including siRNA targeting luciferase were produced in the same manner as in Example 2. A cell line constitutively expressing luciferase was treated with lipid nanoparticles at various concentrations based on siRNA concentration and the degree of inhibition against luciferase activity was evaluated.

To determine the effects of the molecular weight and content of PEG in lipid nanoparticles on efficacy, lipid nanoparticles containing 1, 2, and 5 mol% of PEG2000 (PEG2K-Hz-DSG or PEG2K-PE-DSG) or PEG1000 (PEG1K-Hz-DSG or PEG1K-PE-DSG) were produced. Lipid nanoparticles containing PEG600-Hz-DSG in more various amounts of 1, 2, 5, 10, 20, 30, and 40 mol% were also produced and the efficacy thereof was evaluated.

It can be seen that lipid nanoparticles containing PEG-PE-DSG have no efficacy regardless of the PEG content (FIG. 2A) or have deteriorated efficacy as the content increases (FIG. 2B), whereas PEG-Hz-DSG effectively inhibits luciferase expression regardless of the content. In particular, lipid nanoparticles containing PEG600-Hz-DSG inhibited the expression of luciferase more effectively until the content reached 20 mol%, thereby improving efficacy.

### Example 4: Comparison in cellular uptake and target inhibition efficiency depending on content of PEG in lipid nanoparticles

It is known that, when lipid nanoparticles are produced using PEG-DMG, which is commonly used in the production of lipid nanoparticles, the efficiency of uptake into cells decreases when the PEG content increases. To confirm this, lipid nanoparticles containing 2, 5, and 10 mol% of PEG-DMG were produced doped with 0.2 mol% of a fluorescent dye Dil (1,1'-dioctadecyl-3,3,3',3'-tetramethylindocarbocyanine perchlorate), and then incubated with HepG2 cell line for 24 hours. The cellular uptake efficiency of nanoparticles was analyzed using flow cytometry (FACS). The result showed that cellular uptake decreased as the PEG content increased (FIG. 3, A).

The reduced uptake results in a decrease in the efficacy of lipid nanoparticles. siRNA targeting luciferase was included when lipid nanoparticles containing 0, 0.1, 0.2, 0.5, 1, 2, 5, and 10 mol% of PEG-DMG were produced. Cells constitutively expressing luciferase were treated with the resulting lipid nanoparticles and the activity of luciferase was measured. As can be seen from the graph B of FIG. 3, the efficiency was the best when the PEG content fell within the range of 0.5 to 2 mol%. It can be seen that no efficacy was observed in lipid nanoparticles containing 0 to 0.2 mol% PEG and the inhibitory efficiency against luciferase activity decreases as the PEG content increases in the range of 2 mol% or more. This is because a very low content of PEG may cause nonuniform particles to be formed, whereas PEG above a predetermined amount makes uptake into cells and escape from endosomes difficult.

In order to directly compare the efficacy between lipid nanoparticles containing PEG-DMG and PEG600-Hz-DSG in different amounts, lipid nanoparticles containing the corresponding PEG-lipids in 1, 2, 5, and 10 mol%, respectively and including siRNA targeting luciferase were produced. 24 hours after HEPG2 and HEK293 cell lines constitutively expressing luciferase were treated with the lipid nanoparticles, the luciferase expression level was measured. The result showed that the efficiency of lipid nanoparticles containing PEG-DMG decreased as the PEG content increased, whereas the efficiency of lipid nanoparticles containing PEG600-Hz-DSG did not change or rather improved when the PEG content increased (C, D in FIG. 3). EC₅₀ was calculated based on this data shown in FIGS. 3E and 3F. The EC₅₀ of lipid nanoparticles containing 10% of PEG-DMG and PEG600-Hz-DSG in the HEPG2 cell line were 8.9 nM and 0.2 nM, respectively, the difference between which was about 45-fold, and the EC50 of lipid nanoparticles containing 10% of PEG-DMG and PEG600-Hz-DSG in the HEK293 cell line were 26 nM and 0.72 nM, respectively, the difference between which was about 36-fold.

### Example 5: Confirmation of PEG removal of lipid nanoparticles

It is advantageous in terms of efficiency to remove PEG from lipid nanoparticlesin the endosomes to ensure effective interaction between the endosomal lipid membrane and the lipid nanoparticles after the lipid nanoparticles enter the endosomes of cells. The lipid nanoparticles produced using a general PEG-lipid conjugate having no degradable functional group have a relatively stable structure under acidic conditions that mimic the internal environment of endosomes. On the other hand, lipid nanoparticles containing functional groups that are degraded under acidic conditions, such as PEG-Hz-DSG, become unstable as PEG is removed under acidic conditions. To confirm this, changes in size of nanoparticles containing PEG-DMG in 1.5 mol%, which is a typical lipid nanoparticle production ratio, and lipid nanoparticles containing 10 mol% of PEG600-Hz-DSG used in the present invention were measured in a buffer solution at a pH of 7.4, 5.5, 5.0, or 4.5.

When the change in particle size of nanoparticles produced using PEG-DMG was observed over time under each pH condition, it was found that there was no significant change (A in FIG. 4). On the other hand, the size of lipid nanoparticles containing PEG600-Hz-DSG remained stable at pH 7.4, a neutral condition, but fusion between particles was activated in an acidic pH environment (B in FIG. 4). In addition, it can be seen that the fusion between particles continues over time in each acidic condition and the size gradually increases. In other words, it can be seen that PEG constituting the surface of the nanoparticles is effectively removed under acidic conditions.

Therefore, PEG-Hz-DSG, which contains a functional group that is hydrolyzed under acidic conditions, such as hydrazone, maintains structural stability under acidic conditions when producing lipid nanoparticles (pH 4, ethanol 20 to 40% buffer solution) (FIG. 1), but the same hydrazone was quickly hydrolyzed to separate the PEG and lipid from each other in the acidic buffer condition after the ethanol was removed.

### Example 6: Confirmation of hydrolysis time of degradable functional group

In order to determine whether or not there is a difference in efficacy depending on the timing of removing PEG from the surface of lipid nanoparticles, lipid nanoparticles containing 5 or 10 mol% of PEG600-Hz-DSG were produced using siRNA targeting luciferase. After the lipid nanoparticles were treated with a pH 7 or 4 buffer solution for 24 hours, a cell line constitutively expressing luciferase for 72 hours was treated with the lipid nanoparticles, and then the amount of luciferase expression was measured.

Lipid nanoparticles pretreated with a pH 7 buffer solution could effectively inhibit luciferase expression, but lipid nanoparticles from which PEG was removed in a pH 4 buffer solution had no efficacy at all (FIG. 5). In other words, it was found that PEG must be removed in order to provide efficacy after lipid nanoparticles enter the endosomes of cells, but nanoparticles from which the PEG was removed before uptake into cells lose efficacy thereof.

### Example 7: Evaluation of effects of chemical structure of degradable functional group on efficacy

Differences in hydrolysis half-life under acidic conditions occur depending on the chemical structure of the degradable functional group in the PEG moiety-degradable functional group-lipid conjugate component. To determine the effects of hydrolysis sensitivity under acidic conditions on the efficacy of lipid nanoparticles, lipid nanoparticles containing 1, 2, 5, and 10 mol% of a PEG derivative having ester (PEG600-Ester-DSG, Formula 8) or arylhydrazone (PEG600-PhHz-DSG, Formula 9) as the degradable functional group were produced.

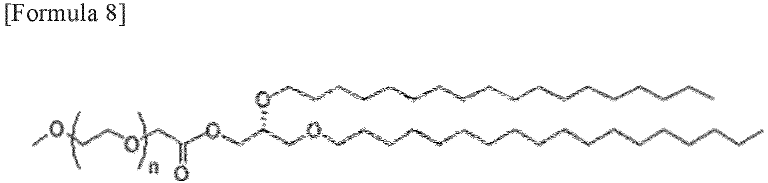

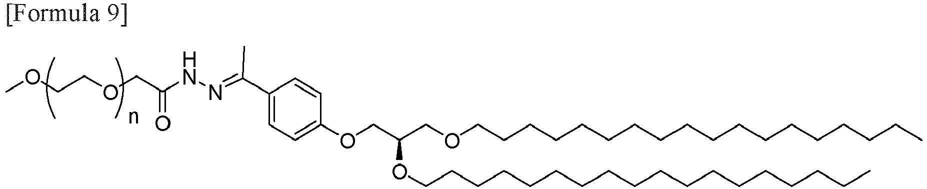

The lipid nanoparticles were produced by incorporating siRNA targeting luciferase in the lipid nanoparticles. A cell line constitutively expressing luciferase was treated with the lipid nanoparticles for 24 hours and then luciferase activity was measured (FIG. 6, A).

It was confirmed that the efficiency of lipid nanoparticles improved as the contents of PEG600-Hz-DSG and PEG600-ester-DSG increased and that the hydrazone functional group had better efficiency than the ester functional group.

To determine the correlation between these results and the sensitivity of each functional group to hydrolysis under acidic conditions, lipid nanoparticles containing 10 mol% of the corresponding PEG moiety-degradable functional group-lipid conjugate were transferred to a PBS pH 7.4 or citric acid pH 4.5 buffer solution, and the change in particle size was measured using dynamic light scattering (FIG. 6, B). The particle size having the corrresponding functional groups increased in the order of hydrazone, ester, and aryl hydrazone, which means that each functional group is easily hydrolyzed under acidic conditions in the order. In other words, it can be seen from A and B of FIG. 6 that as the hydrolysis becomes more active to remove PEG effectivly, the efficiency increases.

### Example 8: Evaluation of lipid membrane fusion and hemolysis induction of lipid nanoparticles

In order to determine the extent to which lipid nanoparticles fuse with the endosomal lipid membrane, liposome lipid membranes or red blood cells were mixed with nanoparticles at pH lower than the pKa of the ionizable lipid, and the lipid membrane fusion or hemolysis was evaluated. The ionizable lipid used herein was DLin-MC3-DMA. The pKa of the amine group of this material is about 6.5 and the amine group has a positive charge at pH 4. The positive charged lipid nanoparticles can be easily fused with the negatively charged lipid membrane of the endosome through electrostatic attraction. However, when PEG is disposed on the surface of the nanoparticle, PEG has a shielding effect that reduces the electrostatic attraction and also has the effect of preventing direct contact between the surface of the nanoparticle and the lipid membrane of the endosome.

In order to evaluate the fusion efficiency of lipid nanoparticles and endosomal lipid membrane, lipid nanoparticles containing 10 mol% of PEG600-Hz-DSG or 1.5 mol% of PEG-DMG, which is commonly used in the lipid nanoparticle production, were produced. Each nanoparticle contained 0.5 mol% of 18:1 NBD PE (1,2-dioleoyl-sn-glycero-3-phosphoethanolamine-N-(7-nitro-2-1,3-benzoxadiazol-4-yl) (ammonium salt)) and 0.5 mol% of 18:1 Liss Rhod PE (1,2-dioleoyl-sn-glycero-3-phosphoethanolamine-N-(lissamine rhodamine B sulfonyl) (ammonium salt)).

Liposomes were produced and used to simulate the endosomal lipid membrane. Soy PS:DOPC:DOPE was dissolved in chloroform at a mol% ratio of 25:25:50 to a total concentration of 1 mM, and the chloroform was removed from the round bottom flask by drying under reduced pressure. 1 mL of PBS was added thereto and sonication was performed to produce liposomes.

Each lipid nanoparticle was mixed with the liposome in a buffer solution of pH 7.4, 6.5, 6.0, 5.5, 5.0, and 4.5. When the two fluorescent lipids, NBD and Liss Rhod, are contained in the lipid nanoparticle, the fluorescence signal cannot be measured because they are close to each other, but when the relative distance between the fluorescent lipids increases as the nanoparticle fuses with the lipid membrane of the liposome, the fluorescence signal of the NBD increases. Based thereon, lipid membrane fusion efficiency can be measured. The lipid membrane fusion efficiency of nanoparticles was determined by measuring the fluorescence signal of NBD under ex 475/em 540 wavelength conditions.

By measuring the generation of fluorescence signals, it was confirmed that lipid nanoparticles containing PEG-DMG or PEG600-Hz-DSG were fused with the lipid membrane of liposomes under acidic conditions (FIG. 7, A). In particular, lipid nanoparticles containing PEG600-Hz-DSG in 10%, which is a higher PEG content than lipid nanoparticles containing PEG-DMG in 1.5%, initiate fusion with the lipid membrane at a relatively higher pH and generate a stronger fluorescence signal. In other words, both the nanoparticles were produced using the same ionizable lipid and thus exhibited the same effect of the positive charge of the ionizable lipid, but PEG in PEG-DMG lipid nanoparticles remained on the particle surface, whereas PEG in PEG600-Hz-DSG lipid nanoparticles was removed by hydrolysis of the hydrazone group and thus exhibited relatively better lipid membrane fusion efficiency.

The fusion efficiency of lipid nanoparticles and lipid membranes can also be determined through hemolysis experiments using red blood cells. FIG. 7B shows the results of the hemolysis-inducing reaction of lipid nanoparticles containing 5 and 10 mol% of PEG-DMG which is commonly used in the production of lipid nanoparticles, under pH 4.5 or 7.4 conditions. It can be seen that the hemolytic reaction is suppressed when the PEG content increases from 5% to 10%. In other words, it can be seen that as the PEG content increases, the lipid membrane fusion capacity is inhibited due to the shielding effect of PEG. On the other hand, for lipid nanoparticles produced using PEG600-Hz-DSG containing a ligand, PEG is removed through hydrolysis under acidic conditions. Therefore, lipid nanoparticles with an increased PEG derivative content of 10% also effectively induce hemolysis.

For reference, it can be seen that under neutral conditions of pH 7.4, both types of lipid nanoparticles do not cause a hemolytic reaction because the amine group of the ionizable lipid is electrically neutral. In other words, when the pH is neutral, there is no interaction with other cells around the lipid nanoparticles, but when the lipid nanoparticles reach the endosomes, the lipid nanoparticles containing PEG derivatives having degradable linker fuse with the endosomal lipid membrane relatively easily.

### Example 9: Relationship between length of lipids constituting PEG moiety-degradable functional group-lipid conjugate and structural stability of lipid nanoparticles

As can be seen from the above examples, PEG surrounding the surface of lipid nanoparticles is necessary for the lipid nanoparticle production process, but has a disadvantage in terms of pharmacological efficacy. Therefore, PEG-DMG, which is a substance in which PEG is linked to a hydrocarbon lipid, myristate having 14 carbon atoms, is usually used as PEG-lipid for lipid nanoparticles, so that PEG-lipid can be easily removed spontaneously from lipid nanoparticles while the lipid nanoparticles circulate in the bloodstream *in vivo.* Lipid nanoparticles produced using the substance with longer fatty acids, such as palmitate (16 carbon atoms) and stearate (18 carbon atoms) cannot allow easy removal of PEG-lipid during circulation through the bloodstream and thus cannot easily enter cell endosomes, and the efficacy thereof is reduced.

However, the method of removing PEG-lipid during the circulation using a PEG-lipid conjugate with a shorter fatty acid is very disadvantageous in terms of the structural stability of lipid nanoparticles. When the PEG-lipid conjugate begins to be separated and removed from the lipid nanoparticle, the stability of the particle itself deteriorates and other lipid components constituting the lipid nanoparticle also begin to be lost. To confirm this, the structural stability was compared between lipid nanoparticles containing 1.5% or 5% of PEG-DMG (lipid carbon length 14) and lipid nanoparticles containing 5% of PEG moiety-degradable functional group-lipid conjugate (Formula 4) having a fatty acid length of 18.

1 mol% of each of fluorescent lipids, 18:1 NBD PE and 18:1 Liss Rhod PE, were used to produce lipid nanoparticles. Lipid nanoparticles were added to rat plasma at 37°C, samples were collected at predetermined times, and the fluorescence intensity of NBD was measured (FIG. 8). When the particles remain stable, the fluorescence signal of the NBD fluorescent dye is weak because the two fluorescent dyes are relatively close together within the nanoparticle. When the nanoparticle structure becomes unstable, it causes the components of the nanoparticle to leak out. In this case, the fluorescent lipids are also released, causing an increase in the fluorescence signal. As can be seen from the data, the lipid nanoparticles produced using PEG-DMG which has a short fatty acid have poor particle stability and the fluorescence signal increases quickly, whereas the PEG moiety-degradable functional group with a long fatty acid had a slow increase in fluorescence signal of lipid conjugates. In other words, in lipid nanoparticles produced intentionally using PEG-lipid conjugates with shorter fatty acids to remove PEG from lipid nanoparticles, the PEG is removed and other lipid components rapidly lost during circulation through the bloodstream. On the other hand, lipid nanoparticles produced using the PEG moiety-degradable functional group-lipid conjugate disclosed herein can maintain a relatively stable structure.

### Example 10: Measurement of immune response induction of lipid nanoparticles

Ionizable lipids that constitute lipid nanoparticles may trigger unnecessary immune responses through interaction with immune cells. In order to minimize this side effect, PEG may be used. As can be seen from Examples above, increasing the PEG content can effectively alleviate the immune response by inhibiting unnecessary interactions, but inevitably seriously inhibits uptake efficiency of nanoparticles by cells and endosomal escape. Therefore, it is difficult to increase the PEG content beyond the minimum required amount.

However, the lipid nanoparticle containing the PEG moiety-degradable functional group-lipid conjugate of the present invention can increase cell uptake efficiency using a ligand and has a chemical functional group that can remove PEG in endosomes. Therefore, when the PEG moiety-degradable functional group-lipid conjugate is used, the induction of an immune response may be inhibited without decreasing the efficacy of the produced lipid nanoparticles even if the PEG content is increased. Therefore, lipid nanoparticles using 10 mol% PEG moiety-degradable functional group-lipid conjugate are mixed with peripheral blood mononuclear cells (PBMC) extracted from rats and the extent to which the lipid nanoparticles induce cytokines is measured. As can be seen from FIG. 9, the degree of inducing immune response of lipid nanoparticles using PEG moiety-degradable functional group-lipid conjugate is similar to that of the group treated with the PBS buffer solution used as a negative control.

### Example 11: Evaluation of efficacy of lipid nanoparticles using animals

Animal experiments were conducted to determine the effectiveness *in vivo* using lipid nanoparticles having efficacy in cell experiments in vitro. For this purpose, lipid nanoparticles containing 5 mol% of PEG2K-PE-DSG or PEG2K-Hz-DSG were produced and each lipid nanoparticle contained siRNA targeting Factor 7. PBS or each lipid nanoparticle was administered into 8-week-old C57BL/6 female mice (Raon Bio) by intravenous injection (I.V. injection) at a dose of 0.3 or 1 mg/kg of each lipid nanoparticle based on siRNA concentration. The volume of the substance administered during injection was maintained at 10 mL/kg. 48 hours after injection, the content of Factor 7 in the serum was measured using the Factor 7 analysis kit (Abcam) and the content was calculated compared to the negative control administered with PBS (FIG. 10).

Similar to the results of experiments using cells (FIG. 2, A), the expression of Factor 7 was inhibited in a dose-dependent manner in lipid nanoparticles containing hydrazone, a degradable functional group, but efficacy could not be observed in lipid nanoparticles that did not contain a degradable functional group.

### Example 12: Evaluation of efficacy of lipid nanoparticles containing ligand

In order to selectively deliver nanoparticles to tissues expressing a specific receptor, lipid nanoparticles can be produced by incorporating a ligand that specifically binds to the corresponding receptor. To determine the effect of lipid nanoparticles containing a ligand, lipid nanoparticles containing 10 mol% of PEG600-Hz-DSG and lipid nanoparticles containing a combination of 0.5 mol% of GalNac-PEG2K-Hz-DSG and 9.5 mol% of PEG600-Hz-DSG for specific delivery to liver tissue were produced. The nanoparticles were administered to mice at a dose of 0.1 mg/kg based on siRNA concentration, and animal experiments were conducted in the same manner as in Example 11.

The result showed that lipid nanoparticles containing a liver-specific ligand exhibited superior efficacy compared to lipid nanoparticles contain no ligand (FIG. 11).

### Example 13: Evaluation of cytotoxicity of lipid nanoparticles

The relative toxicity of lipid nanoparticles containing commonly used PEG-DMG and lipid nanoparticles containing PEG moiety-degradable functional group-lipid conjugate used in the present invention were evaluated. For this purpose, lipid nanoparticles containing 1.5 mol% of PEG-DMG and lipid nanoparticles containing a mixture of 1 mol% of GalNAc-PEG2K-Hz-DSG and 9 mol% of PEG600-Hz-DSG, respectively, were produced. Cell lines such as HepG2, HEK293, H460, and A549 were treated with lipid nanoparticles based on the siRNA concentration in the lipid nanoparticles. For 72 hours, the viability of each cell line was measured using the CellTiter-Glo kit (Promega) (FIG. 12).

Treatment of cells with high concentrations of lipid nanoparticles containing PEG-DMG caused death of a number of cells, whereas lipid nanoparticles containing 10 mol% of the PEG moiety-degradable functional group-lipid conjugate used in the present invention did not cause cell death, which indicates that the lipid nanoparticles had very low toxicity.

The therapeutic agent using lipid nanoparticles according to the present invention minimizes *in vivo* side effects caused by the components of the nanoparticles, effectively delivers the nanoparticles to target cells, and transports efficiently pharmacological substances such as nucleic acids from endosomes into the cytoplasm in the target cells.

Lipid nanoparticles according to the present invention can suppress non-specific interactions because the outside of the particles is coated with PEG. Further, by attaching a ligand to the end of PEG, the lipid nanolparticle can be delivered only to the target cells. Lipid nanoparticles delivered into and encapsulated in endosomes by ligands and cell receptors can increase the interaction between the surface of lipid nanoparticles and the endosomal lipid membrane because the degradable functional group is degraded within the endosome and PEG outside the nanoparticle is removed. In addition, since relatively long-chain fatty acids can be employed to constitute the lipid part of the PEG-lipid, structural stability is imparted to the lipid nanoparticles, so the lipid nanoparticles can be stable during treatment with the particles.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

## Claims

1. A lipid nanoparticle comprising:
(a) a lipid formulations containing an ionizable lipid and a polyethylene glycol moiety (PEG moiety)-degradable functional group-lipid conjugate; and
(b) a drug, nucleic acid, or combination thereof encapsulated in the lipid formulations.

2. The lipid nanoparticle of claim 1 wherein the PEG moiety-degradable functional group-lipid conjugate is represented by Formula 1: 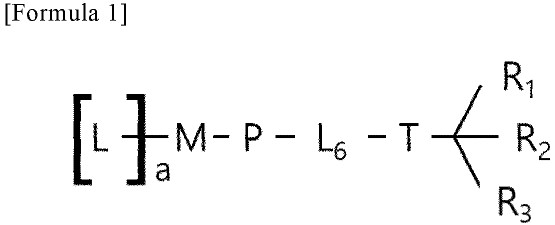 wherein
a is 0 or 1;
L is a targeting ligand;
M is H, OH, single bond, O, S, C(O), NHC(O), C(O)NH, OC(O) or C(O)O;
P is CH₂O(CH₂CH₂O)_{q}CH₂ or CH₂CH₂O(CH₂CH₂O)_{q}CH₂, in which q is an integer of 2 to 120;
L₆ is C(O)NH-N=CR₄, R₄C=N-NHC(O), NH-N=CR₄, R₄C=N-NH, C(O)O, OC(O), OC(O)O, O-N=CR₄, R₄C=N-O, S-S, S, trans-cyclooctene, or 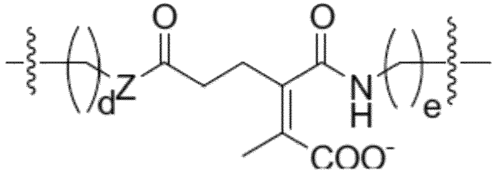
in which, R₄ is H, C₁₋C₂₀ alkyl, C₂₋C₂₀ alkenyl, C₂₋C₂₀ alkynyl, C₃₋C₁₀ cycloalkyl, C₆₋ C₂₀ aryl or a heterocycle, which is a radical containing a heteroatom selected from fluorine, oxygen, sulfur, and nitrogen, Z is NH, O or S, d is an integer of 1 to 10, and e is an integer of 1 to 10;
T is a single bond or 1,4-C₆H₄O-; and
R₁ and R₃ are each independently -Y-R, R₂ is -CH₂-Y-R, in which Y is a single bond, O, S, C(O), C(O)O, OC(O), C(O)NH, or NHC(O), and R is H, C₁₀-C₂₀ alkyl, alkenyl or sterol.

3. The lipid nanoparticle of claim 2 wherein the targeting ligand L is represented by Formula 2: 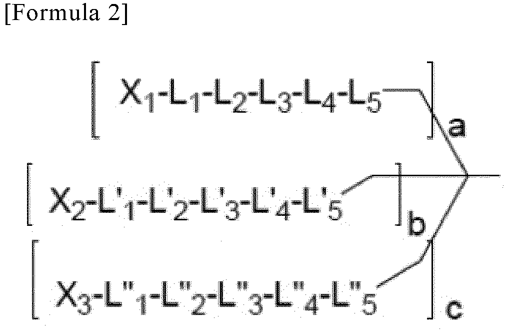 wherein
a, b and c are 0 or 1, with the proviso that at least one of a, b or c is 1;
X₁, X₂ and X₃ are targeting ligands;
L₁, L'₁ and L"₁ are a single bond, O, S, C(O), NHC(O), C(O)NH, OC(O) or C(O)O;
L₂, L'₂ and L"₂ are (CH₂)ₙ or (OCH₂CH₂)ₘ, in which n is an integer of 1 to 20 and m is an integer of 1 to 10;
L₃, L'₃ and L"₃ are a single bond, O, S, C(O), NHC(O), C(O)NH, OC(O) or C(O)O;
L₄, L'₄ and L'₄ are (CH₂)ₙ, in which n is an integer of 1 to 20; and
L₅, L'₅ and L"₅ are a single bond, O, S, C(O), NHC(O), C(O)NH, OC(O) or C(O)O.

4. The lipid nanoparticle of claim 3 wherein X₁, X₂, and X₃ are selected from the group consisting of N-acetyl-D-galactosamine (GalNAc), N-acetyl-D-galactose, D-galactose, N-acetyl-D-glucosamine, N-acetyl-D-glucosamine, D-glucose, D-mannose, L-fucose, carbohydrate derivatives, folate, transferrin, RGD peptides, cyclic RGD peptides, TAT peptides, R9 peptides, CADY peptides, HA2 peptides, monoclonal antibodies, antigen-binding fragments or antibody fragments, single-chain variable fragment (scFv) and aptamers.

5. The lipid nanoparticle of claim 1 wherein the polyethylene glycol (PEG) moiety-degradable functional group-lipid conjugate is represented by Formula 3: 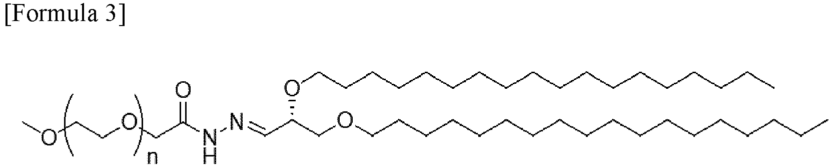 wherein n is an integer of 2 to 120.

6. The lipid nanoparticle of claim 1 wherein the polyethylene glycol (PEG) moiety-degradable functional group-lipid conjugate is represented by Formula 4: 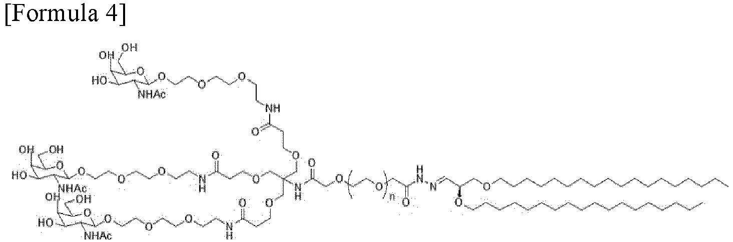 wherein n is an integer of 2 to 120.

7. The lipid nanoparticle of claim 2 wherein the polyethylene glycol (PEG) moiety-degradable functional group-lipid conjugate is a mixture of a compound of Formula 1 wherein a is 0 and a compound of Formula 1 wherein a is 1.

8. The lipid nanoparticle of claim 7 wherein a molar ratio of the compound of in Formula 1 wherein a is 0 to the compound of in Formula 1 wherein a is 1 is 0.01 to 99.9:0.01 to 99.9.

9. The lipid nanoparticle of claim 1 wherein a content of the ligand-PEG moiety-degradable functional group-lipid conjugate in the lipid nanoparticle is 0.5 to 50 mol% based on a total lipid constituting the lipid nanoparticle.

10. The lipid nanoparticle of claim 1 wherein the lipid nanoparticle has a size of 20 to 200 nm.

11. The lipid nanoparticle of claim 1 wherein the ionizable lipid comprises at least one selected from (6Z, 9Z, 28Z, 31Z)-heptatriaconta 6,9,28,31-tetraen-19-yl 4-(dimethylamino)butanoate (DLin-MC3-DMA), [(4-hydroxybutyl)azanediyl]di(hexan-6,1-diyl)bis(2-hexyldecanoate) (ALC-0315), 8-[(2-hydroxyethyl)[6-oxo-6-(undecyloxy)hexyl]amino]-octanoic acid (SM-102), 1-linoleoyl-2-linoleyloxy-3-dimethylaminopropane (DLin-2-DMAP), 1,2-dilinoleyl carbamoyloxy-3-dimethylaminopropane (DLin-C-DAP), 1,2-dilinoleoyl-3-dimethylaminopropane (DLin-DAP), 1,2-dilinoleyloxy-N,N-dimethylaminopropane (DLin-DMA), 1,2-dilinoleyl-4-dimethylaminomethyl-[1,3]-dioxolane (DLin-K-DMA), 2,2-dilinoleyl-4-(2-dimethylaminoethyl)-[1,3]-dioxolane (DLin-KC2-DMA), 1,2-dioleoyl-3-dimethylammonium propane (DODAP), N,N-dimethyl-(2,3-dioleyloxy)propylamine (DODMA), dioctadecylamidoglycyl-spermine (DOGS), spermine cholesteryl carbamate (GL-67), bis-guanidinium-spermidine-cholesterol (BGTC), 3*β*-(N-(N',N'-dimethylaminoethane)-carbamoyl) cholesterol (DC-Chol), 1,1'-(2-(4-(2-((2-(bis(2-hydroxydecyl)amino)ethyl)(2-hydroxydecyl)amino)ethyl)piperazin-1-yl)ethyazandiyl)didodecan-2-ol (C12-200), N-t-butyl-N'-tetradecylamino-propionamidine (diC14-amidine), dimethyl dioctadecyl ammonium bromide (DDAB), N-(1,2-dimyristyl oxyprop-3-yl)-N,N-dimethyl-N-hydroxyethylammonium bromide (DMRIE), N,N-dioleyl-N,N-dimethylammonium chloride (DODAC), dioleyloxypropyl-3-dimethylhydroxyethylammonium bromide (DORIE), N-(1-(2,3-dioleyloxyl)propyl)-N-2-(sperminecarboxamid)ethyl)-N,N-dimethylammonium trifluoroacetate (DOSPA), 1,2-dioleoyl trimethylammonium propane chloride (DOTAP), N-(1-(2,3-dioleyloxy)propyl)-N,N, N-trimethylammonium chloride (DOTMA) and aminopropyl-dimethyl-bis(dodecyloxy)-propane amidium bromide (GAP-DLRIE).

12. The lipid nanoparticle of claim 1 further comprising sterol lipid and neutral lipid.

13. The lipid nanoparticle of claim 12, wherein the sterol lipid is cholesterol or cholesteryl ester.

14. The lipid nanoparticle of claim 12, wherein the neutral lipid is phospholipid or sphingolipid.

15. The lipid nanoparticle of claim 14 wherein the phospholipid is selected from the group consisting of DOPE (dioleoylphosphatidylethanolamine), DSPC (distearoylphosphatidylcholine), POPC (palmitoyloleoylphosphatidylcholine), EPC (egg phosphatidylcholine), DOPC (dioleoylphosphatidylcholine), DPPC (dipalmitoylphosphatidylcholine), DOPG (dioleoylphosphatidylglycerol), DPPG (dipalmitoylphosphatidylglycerol), DSPE (distearoylphosphatidylethanolamine), PE (Phosphatidylethanolamine), DPPE (dipalmitoylphosphatidylethanolamine), DOPE (1,2-dioleoyl-sn-glycero-3-phosphoethanolamine), POPE (1-palmitoyl-2-oleoyl-sn-glycero-3-phosphoethanolamine), POPC (1-palmitoyl-2-oleoyl-sn-glycero-3-phosphocholine), DOPS (1,2-dioleoyl-sn-glycero-3-[phospho-L-serine]), ceramide, and sphingomyelin.

16. The lipid nanoparticle of claim 1 wherein the drug comprises at least one selected from the group consisting of peptides, protein drugs, protein-nucleic acid structures, and anionic biopolymer-drug conjugates.

17. The lipid nanoparticle of claim 16 wherein the nucleic acid comprises at least one selected from the group consisting of single-stranded siRNA, double-stranded siRNA, rRNA, DNA, cDNA, plasmids, aptamers, mRNA, tRNA, lncRNA, piRNA, circRNA, saRNA, antisense oligonucleotides, shRNA, miRNA, ribozyme, PNA, and DNAzyme.

18. A method of producing the lipid nanoparticle of claim 1 comprising:
(a) mixing an organic solution containing a lipid formulations containing an ionizable lipid and a polyethylene glycol moiety (PEG moiety)-degradable functional group-lipid conjugate, with a buffer solution containing a drug, nucleic acid, or combination thereof and adjusting pH; and
(b) removing a solvent from the solution.

19. The method of claim 18 wherein a mix ratio of the organic solution to the buffer solution is 1:1 to 1:100 on a basis of volume.

20. The method of claim 18 wherein the PEG moiety-degradable functional group-lipid conjugate is represented by Formula 1: 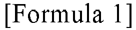 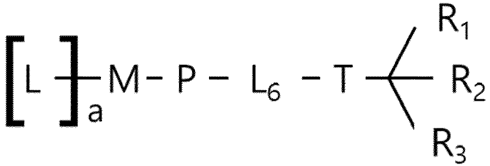 wherein
a is 0 or 1;
L is a targeting ligand;
M is H, OH, single bond, O, S, C(O), NHC(O), C(O)NH, OC(O) or C(O)O;
P is CH₂O(CH₂CH₂O)_{q}CH₂ or CH₂CH₂O(CH₂CH₂O)_{q}CH₂, in which q is an integer of 2 to 120;
L₆ is C(O)NH-N=CR₄, R₄C=N-NHC(O), NH-N=CR₄, R₄C=N-NH, C(O)O, OC(O), OC(O)O, O-N=CR₄, R₄C=N-O, S-S, S, *trans*-cyclooctene, or 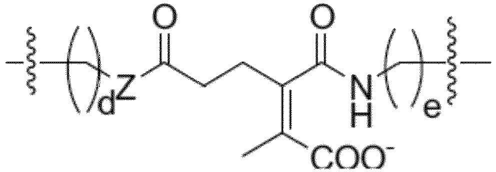
in which, R₄ is H, C₁₋C₂₀ alkyl, C₂₋C₂₀ alkenyl, C₂₋C₂₀ alkynyl, C₃₋C₁₀ cycloalkyl, C₆₋ C₂₀ aryl or a heterocycle, which is a radical containing a heteroatom selected from fluorine, oxygen, sulfur, and nitrogen, Z is NH, O or S, d is an integer of 1 to 10, and e is an integer of 1 to 10;
T is a single bond or 1,4-C₆H₄O-; and
R₁ and R₃ are each independently -Y-R, R₂ is -CH₂-Y-R, in which Y is a single bond, O, S, C(O), C(O)O, OC(O), C(O)NH, or NHC(O), and R is H, C₁₀-C₂₀ alkyl, alkenyl or sterol.

21. The method of claim 20 wherein the targeting ligand L is represented by Formula 2: 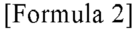 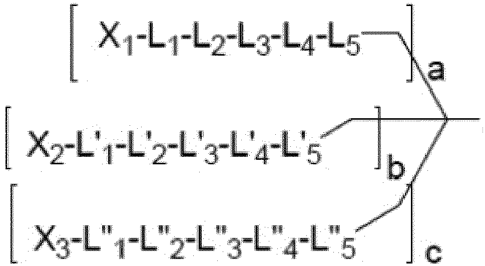 wherein
a, b and c are 0 or 1, with the proviso that at least one of a, b or c is 1;
X₁, X₂ and X₃ are targeting ligands;
L₁, L'₁ and L₁ are a single bond, O, S, C(O), NHC(O), C(O)NH, OC(O) or C(O)O;
L₂, L'₂ and L"₂ are (CH₂)ₙ or (OCH₂CH₂)ₘ, in which n is an integer of 1 to 20 and m is an integer of 1 to 10;
L₃, L'₃ and L"₃ are a single bond, O, S, C(O), NHC(O), C(O)NH, OC(O) or C(O)O;
L₄, L'₄ and L"₄ are (CH₂)ₙ, in which n is an integer of 1 to 20; and
L₅, L'₅ and L"₅ are a single bond, O, S, C(O), NHC(O), C(O)NH, OC(O) or C(O)O.

22. The method of claim 21 wherein X₁, X₂, and X₃ are selected from the group consisting of N-acetyl-D-galactosamine (GalNAc), N-acetyl-D-galactose, D-galactose, N-acetyl-D-glucosamine, N-acetyl-D-glucosamine, D-glucose, D-mannose, L-fucose, carbohydrate derivatives, folate, transferrin, RGD peptides, cyclic RGD peptides, TAT peptides, R9 peptides, CADY peptides, HA2 peptides, monoclonal antibodies, antigen-binding fragments or antibody fragments, single-chain variable fragment (scFv) and aptamers.

23. The method of claim 18 wherein the polyethylene glycol (PEG) moiety-degradable functional group-lipid conjugate is represented by Formula 3:
[Formula 3] 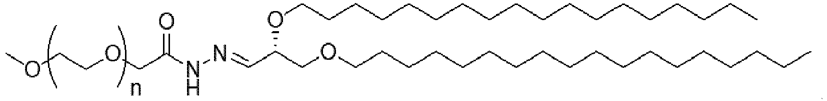 wherein n is an integer of 2 to 120.

24. The method of claim 18 wherein the PEG moiety-degradable functional group-lipid conjugate is represented by Formula 4: 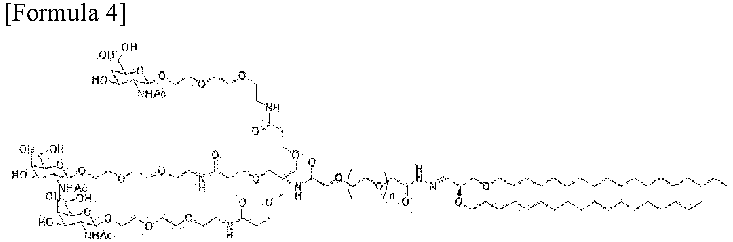 wherein n is an integer of 2 to 120.

25. The method of claim 20 wherein the PEG moiety-degradable functional group-lipid conjugate is a mixture of a compound of Formula 1 wherein a is 0 and a compound of Formula 1 wherein a is 1.

26. The method of claim 25 wherein a molar ratio of the compound of in Formula 1 wherein a is 0 to the compound of in Formula 1 wherein a is 1 is 0.01 to 99.9:0.01 to 99.9.

27. The method of claim 18 wherein a content of the ligand-PEG moiety-degradable functional group-lipid conjugate in the lipid nanoparticle is 0.5 to 50 mol% based on a total lipid constituting the lipid nanoparticle.

28. The method of claim 18 wherein the lipid nanoparticle has a size of 20 to 200 nm.

29. The method of claim 18 wherein the ionizable lipid comprises at least one selected from (6Z, 9Z, 28Z, 31Z)-heptatriaconta 6,9,28,31-tetraen-19-yl 4-(dimethylamino)butanoate (DLin-MC3-DMA), [(4-hydroxybutyl)azanediyl]di(hexan-6,1-diyl)bis(2-hexyldecanoate) (ALC-0315), 8-[(2-hydroxyethyl)[6-oxo-6-(undecyloxy)hexyl]amino]-octanoic acid (SM-102), 1-linoleoyl-2-linoleyloxy-3-dimethylaminopropane (DLin-2-DMAP), 1,2-dilinoleyl carbamoyloxy-3-dimethylaminopropane (DLin-C-DAP), 1,2-dilinoleoyl-3-dimethylaminopropane (DLin-DAP), 1,2-dilinoleyloxy-N,N-dimethylaminopropane (DLin-DMA), 1,2-dilinoleyl-4-dimethylaminomethyl-[1,3]-dioxolane (DLin-K-DMA), 2,2-dilinoleyl-4-(2-dimethylaminoethyl)-[1,3]-dioxolane (DLin-KC2-DMA), 1,2-dioleoyl-3-dimethylammonium propane (DODAP), N,N-dimethyl-(2,3-dioleyloxy)propylamine (DODMA), dioctadecylamidoglycyl-spermine (DOGS), spermine cholesteryl carbamate (GL-67), bis-guanidinium-spermidine-cholesterol (BGTC), 3*β-*(N-(N',N'-dimethylaminoethane)-carbamoyl) cholesterol (DC-Chol), 1,1'-(2-(4-(2-((2-(bis(2-hydroxydecyl)amino)ethyl)(2-hydroxydecyl)amino)ethyl)piperazin-1-yl)ethyazandiyl)didodecan-2-ol (C12-200), N-t-butyl-N'-tetradecylamino-propionamidine (diC14-amidine), dimethyl dioctadecyl ammonium bromide (DDAB), N-(1,2-dimyristyl oxyprop-3-yl)-N,N-dimethyl-N-hydroxyethylammonium bromide (DMRIE), N,N-dioleyl-N,N-dimethylammonium chloride (DODAC), dioleyloxypropyl-3-dimethylhydroxyethylammonium bromide (DORIE), N-(1-(2,3-dioleyloxyl)propyl)-N-2-(sperminecarboxamid)ethyl)-N,N-dimethylammonium trifluoroacetate (DOSPA), 1,2-dioleoyl trimethylammonium propane chloride (DOTAP), N-(1-(2,3-dioleyloxy)propyl)-N,N, N-trimethylammonium chloride (DOTMA) and aminopropyl-dimethyl-bis(dodecyloxy)-propane amidium bromide (GAP-DLRIE).

30. The method of claim 18 further comprising sterol lipid and neutral lipid.

31. The method of claim 30 wherein the sterol lipid is cholesterol or cholesteryl ester.

32. The method of claim 30 wherein the neutral lipid is phospholipid.

33. The method of claim 32 wherein the phospholipid is selected from the group consisting of DOPE (dioleoylphosphatidylethanolamine), DSPC (distearoylphosphatidylcholine), POPC (palmitoyloleoylphosphatidylcholine), EPC (egg phosphatidylcholine), DOPC (dioleoylphosphatidylcholine), DPPC (dipalmitoylphosphatidylcholine), DOPG (dioleoylphosphatidylglycerol), DPPG (dipalmitoylphosphatidylglycerol), DSPE (distearoylphosphatidylethanolamine), PE (Phosphatidylethanolamine), DPPE (dipalmitoylphosphatidylethanolamine), DOPE (1,2-dioleoyl-sn-glycero-3-phosphoethanolamine), POPE (1-palmitoyl-2-oleoyl-sn-glycero-3-phosphoethanolamine), POPC (1-palmitoyl-2-oleoyl-sn-glycero-3-phosphocholine), DOPS (1,2-dioleoyl-sn-glycero-3-[phospho-L-serine]), ceramide, and sphingomyelin.

34. The method of claim 18 wherein the drug comprises at least one selected from the group consisting of peptides, protein drugs, protein-nucleic acid structures, and anionic biopolymer-drug conjugates.

35. The method of claim 34 wherein the nucleic acid comprises at least one selected from the group consisting of single-stranded siRNA, double-stranded siRNA, rRNA, DNA, cDNA, plasmids, aptamers, mRNA, tRNA, lncRNA, piRNA, circRNA, saRNA, antisense oligonucleotides, shRNA, miRNA, ribozyme, PNA, and DNAzyme.
